# EUROPEAN PATENT APPLICATION

(11) **EP 3 357 423 A1**
(43) Date of publication of application: **08.08.2018**
(21) Application number: 16851196.2
(22) Date of filing: 15.09.2016
(51) Int. Cl.: A61B 5/16, A61B 5/02, A61B 5/0476, A61B 5/18

(54) **BIOLOGICAL STATE ESTIMATION DEVICE, BIOLOGICAL STATE ESTIMATION METHOD, AND COMPUTER PROGRAM**

(30) Priority: 28.09.2015 JP 2015190495; 11.12.2015 JP 2015242479
(71) Applicant: Delta Kogyo Co., Ltd., Aki-gun, Hiroshima 735-8501 (JP)
(72) Inventor: FUJITA, Etsunori, Aki-gun, Hiroshima 735-8501 (JP); HORIKAWA, Masahiro, Aki-gun, Hiroshima 735-8501 (JP)
(74) Representative: Hoffmann Eitle
(86) International application number: PCT/JP2016/077274
(87) International publication number: WO 2017/057022

(57) **Abstract**

A basic physical condition of a person is estimated. The present invention calculates a plurality of indexes indicating variations of states of bioregulation function elements, including an index ascribable to fluctuation highly correlated with a brain function, an autonomic nervous function, bodily and mental fatigue, or sensation, which are obtained from a bioregulation function element judging means 200, every predetermined judgment times set for the respective bioregulation function elements in advance to find time-series variations of the indexes. A basic physical condition estimating means 300 analyzes the time-series variations in order of priorities. Consequently, it is possible to find a physical condition from fluctuation performance serving as a basis of a regulation system of a person in a basic physical condition estimation time which is set longer than the judgment times of the respective bioregulation function elements.

## Description

### Technical Field

The present invention relates to a biological state estimation device, a biological state estimation method, and a computer program for estimating a basic physical condition of a person based on a biosignal measured from the person by a biosignal measurement device.

### Background Art

In Patent Documents 1 to 5 and so on, the present inventors have proposed an art to detect vibration generated on the body surface of the back in the upper body of a person by a biosignal measurement device and analyze the state of the person. Sound and vibration information arising from motions of the heart and the aorta, which is detected from the back of the upper body of the person, is pressure vibration arising from the motions of the heart and the aorta, and includes information on the systole and diastole of the ventricles, information on vascular wall elasticity which serves as an auxiliary pump for the circulation, and information on reflected waves. That is, the sound and vibration information includes information on a back body surface pulse wave (including an aortic pulse wave (APW)) of around 1 Hz generated on the back surface due to the motions of the heart and the aorta and information on sound conveyed to the back side in accordance with heartbeat ("pseudo heart sound" (in this specification, sound of the heart collected from the back side is referred to as "pseudo heart sound", in contrast to heart sound which is sound of the heart collected from the chest side). A signal waveform accompanying heart rate variability includes information on neural activities of the sympathetic nervous system and the parasympathetic nervous system, and a signal waveform accompanying aortic oscillation includes information on sympathetic nerve activity.

In Patent Document 1, slide calculation is performed in which a predetermined time width is set in a time-series waveform of back body surface pulse waves (APW) of around 1 Hz extracted from collected biosignals (sound and vibration information), to find a frequency gradient time-series waveform, and according to the tendency of its variation, for example, according to whether its amplitude is on the decrease or the increase, a biological state is estimated. It is also disclosed that, by frequency analysis of the biosignal, power spectra of respective frequencies corresponding to predetermined signals such as a function regulation signal, a fatigue reception signal, and an activity regulation signal belonging to a ULF band (ultra low-frequency band) to a VLF band (very low-frequency band) are found, and the state of a person is judged from time-series variations of the respective power spectra. Since the fatigue reception signal indicates a progress degree of fatigue in a normal active state, additionally comparing predominant degrees of the power spectra of the function regulation signal and the activity regulation signal makes it possible to judge the state of a person (a sympathetic nerve predominant state, a parasympathetic nerve predominant state, or the like). It is further disclosed that, with the total value of the power spectra of frequency components corresponding to these three signals being set as 100, time-series distribution ratios of the respective frequency components are found, and the state of a person is judged using time-series variations of the distribution ratios.

As a method of quantifying a biological state, Patent Document 2 proposes an art to represent the biological state as a physical condition map and a sensation map. To create them, the aforesaid APW is frequency-analyzed, an analyzed waveform in each target analysis section is displayed on log-log axes, the analyzed waveform is classified into a low-frequency band, an intermediate-frequency band, or a high-frequency band, and according to a gradient of the classified analyzed waveform and the shape of the whole analyzed waveform, the analyzed waveform is scored based on a predetermined criterion, and the results are plotted on coordinate axes. The physical condition map shows the control state of the autonomic nervous system from a viewpoint of the balance between the sympathetic nerves and the parasympathetic nerves, and in the sensation map, the state of a change of heart rate variability is superimposed on the physical condition map.

Patent Documents 3 to 5 disclose a means for judging a homeostasis function level. For the judgment, the means for judging the homeostasis function level uses at least one or more of plus/minus of a differentiated waveform of a frequency gradient time-series waveform, plus/minus of an integrated waveform obtained by integrating the frequency gradient time-series waveform, absolute values of frequency gradient time-series waveforms obtained by absolute value processing of a frequency gradient time-series waveform found by a zero-cross method and a frequency gradient time-series waveform found by a peak detection method, and so on. By using the combination of these, it is found on which level the homeostasis function is. For example, the level can be set such that, when the frequency gradient and the integrated value are used and they are predetermined values or more, it is judged that "the homeostasis function level is 1", or when the differential value is equal to or less than a predetermined value and "peak is predominant" out of the two absolute values, it is judged that "the homeostasis function level is 4". The combination of these, a threshold value for the judgment, and so on are decided based on the results of statistical processing of data of many subjects. Further, the homeostasis function level is divided into, for example, five to seven stages, and the homeostasis function level is judged superior (high concentration degree, or the like) to inferior (excessively tense state, low concentration because of inattentive driving, and the like). It is also disclosed that the setting for monitor display is made such that the levels in the five to seven stages are displayed with letters, or in cases where the level is intermediate (normal) or higher, it is collectively judged that the homeostasis function is superior, and in cases where the level is lower, it is collectively judged that the homeostasis function is inferior, and different colors are displayed on the monitor for the respective levels.

Non-patent Document 1 discloses an art to find, regarding finger plethysmogram information, a frequency gradient time-series waveform of a power value reflecting information on the sympathetic nerve, and plot integrated values resulting from absolute value processing of this, in a time-series manner as a fatigue degree to depict a fatigue curve, from which muscle fatigue is found. Non-patent Document 2 discloses an art to arithmetically process biosignals which are obtained from the back of a person using an air pack sensor, to depict a fatigue curve by a similar method and grasp muscle fatigue. That is, it is possible to grasp the state of the muscle fatigue by using a frequency gradient time-series waveform (a frequency gradient time-series waveform by a zero-cross method in the case of APW) of a power value reflecting information on the sympathetic nerve.

### Prior Art Document

### Patent Document

Patent Document 1: Japanese Patent Application Laid-open No. 2011-167362
Patent Document 2: Japanese Patent Application Laid-open No. 2012-239480
Patent Document 3: WO2011/046178
Patent Document 4: Japanese Patent Application Laid-open No. 2014-117425
Patent Document 5: Japanese Patent Application Laid-open No. 2014-223271

### Non-Patent Document

Non-patent Document 1: "Development of simplified appraisal method of fatigue on sitting for extended periods by the data of finger plethysmogram", Etsunori FUJITA (three others), Ergonomics Vol. 40, No. 5 ('04)
Non-patent Document 2: "Application of biological fluctuation signals measured by non-invasive sensor to fatigue and hypnagogia prediction", Naoki OCHIAI (six others), The 39th Japan Ergonomics Society, Chugoku-Shikoku Branch Conference, Proceedings, issued on November 25, 2006, publishing office: Japan Ergonomics Society Chugoku-Shikoku branch office

### Summary of the Invention

### Problems to Be Solved by the Invention

All of the above-described arts judge the state of a person by analyzing elements ascribable to fluctuations of the bioregulation functions, but they differ in the arithmetic processing of the biosignal, and the output judgment result is the timing of a hypnagogic symptom, the fatigue degree, or the change of the homeostasis function level and differs depending on the aim. However, the judgment results are output separately. Patent Document 4 also discloses an art in which one device judges time-series variations of a plurality of indexes relevant to elements ascribable to fluctuations of bioregulation functions, such as a hypnagogic symptom phenomenon, an imminent sleep phenomenon, a low consciousness traveling state, a homeostasis function level, an initial fatigue state, and a feeling judgment, and outputs the judgment results to one monitor, but similarly to the above, the time-series variations of the respective indexes are judged separately.

Finding various indexes as in the art disclosed in Patent Document 4 is, of course, very important for more accurately grasping the state of a person, in particular, the state of a driver, but since the above-described conventional art basically uses indexes relevant to the autonomic nervous system and puts emphasis on more accurately outputting the states of a person corresponding to these indexes, the extraction time (judgment time) of the biosignal being a judgment target is relatively short, and the judgment result is output at a frequency of once per several ten seconds to several minutes. However, there is also a demand by, for example, a driver or a manager monitoring the information from a distant place for roughly grasping a basic physical condition over a relatively long time span such as thirty minutes or one hour (a predominant, typical, or average physical condition in this period of time) during driving. Specifically, if a driver himself/herself can roughly infer whether, after, for example, one-hour driving, he/she basically has been driving under a good physical condition during this period, or whether he/she has been driving under a poor physical condition, being assisted by a bioregulation function that maintains homeostasis, he/she can be promoted to decide himself/herself to, for example, take a rest early.

The present invention was made in consideration of the above points, and has an object to provide a biological state estimation device, a biological state estimation method, and a computer program which make it possible for a driver or the like to more easily grasp his/her rough physical condition, by using a plurality of pieces of information on time-series variations of elements (bioregulation function elements) ascribable to fluctuations of respective bioregulation functions on which the judgment is successively made in a relatively short time, processing these pieces of information under a predetermined condition, and outputting a basic physical condition of a person during a basic physical condition estimation time set longer.

### MEANS FOR SOLVING THE PROBLEMS

As a result of studious studies with the aim to solve the above problems, the present inventors have completed the present invention, focusing on the following points. Specifically, sound and vibration information arising from the motions of the heart and the aorta, which is detected from the back of the upper body of a person, in particular, a back body surface pulse wave (APW) of around 1 Hz included in the sound and vibration information includes information on elasticity of blood vessels, information on reflected waves, and so on. Therefore, by analyzing the back body surface pulse wave (APW) to find a later-described frequency gradient time-series waveform, it is possible to find information on the total biological fluctuation which is a bioregulation function element. When a variation state of each frequency is found in the fluctuation information, the way a distribution ratio of each frequency varies reflects a fluctuation element of a brain wave (*θ* wave, *α* wave, *β* wave), and accordingly, by an analysis using the time-series waveforms of the distribution ratios, it is possible to get fluctuation information indicating, for example, in which frequency band of the brain wave, the fluctuation is predominant. Further, a frequency gradient time-series waveform found by a zero-cross method is governed by the autonomic nervous system but in its portion reflecting the fluctuation of the brain wave, the frequency gradient time-series waveform by the zero-cross method is frequency-analyzed, and the result is expressed by a power spectrum ratio of three frequency components, namely, a function regulation signal represented by 0.0017 Hz, a fatigue reception signal represented by 0.0035 Hz, and an activation regulation signal represented by 0.0053 Hz, and in the distribution ratios of the three frequency components expressing the form of, what is called, the frequency-analyzed power spectra, a portion where the distribution ratio abruptly changes is thought to be regarded as a portion indicating the manifestation of a function of the endocrine system rather than an autonomic nervous reaction. Further, the frequency gradient time-series waveform found by the zero-cross method, when subjected to absolute value processing, indicates a degree of the manifestation of the sympathetic nerves, while a frequency gradient time-series waveform found by a peak detection method indicates a degree of the manifestation of the parasympathetic nerves. Therefore, by using these, it is possible to grasp the state of the manifestation of the bioregulation functions in more detail. For example, the frequency gradient time-series waveform found by the zero-cross method is subjected to the absolute value processing, and the results are integrated, whereby a fatigue curve indicating a fatigue degree of a person is found and the state of muscle fatigue can be grasped. Further, by using at least one of plus/minus of a differentiated waveform of the frequency gradient time-series waveform found by the zero-cross method, absolute values of the frequency gradient time-series waveform found by the zero-cross method or the peak detection method, and so on, it is possible to grasp the state of variation of a homeostasis function level. The fatigue curve and a time-series waveform of the homeostasis function level also derive from the frequency gradient time-series waveform and serve as indexes reflecting information on the fluctuations of the autonomic nervous system, the brain function, and so on. In addition, indexes highly correlated with bodily and mental fatigue (physical condition map, sensation map), and indexes highly correlated with a sensation (sensation with which fatigue found from the appearance frequency of a level corresponding to caution or warning out of the homeostasis function levels is perceived, or the frequency of the occurrence of lassitude feeling or the like) are also found. They indicate the fluctuation states of the functions of the brain, the autonomic nervous system, and the endocrine system from a plurality of viewpoints, and statistically processing the indexes of these plural kinds of fluctuations as described later is thought to make it possible to estimate what basic physical condition an analysis target person has.

Specifically, a biological state estimation device of the present invention is a biological state estimation device which analyzes a biosignal measured from a person by a biosignal measurement device and estimates a biological state, the biological state estimation device including: a bioregulation function element judging means which analyzes the biosignal and calculates a plurality of indexes indicating variations of states of bioregulation function elements, including an index ascribable to fluctuation highly correlated with a brain function, an autonomic nervous function, bodily and mental fatigue, or sensation, every predetermined judgment times set for the respective bioregulation function elements in advance to find time-series variations of the indexes; a basic physical condition estimating means which estimates a basic physical condition of the person in a basic physical condition estimation time set longer than the judgment times of the bioregulation function elements in the bioregulation function element judging means; and a basic physical condition outputting means which outputs a level of the basic physical condition of the person estimated by the basic physical condition estimating means, every time the predetermined basic physical condition estimation time passes, wherein the basic physical condition estimating means estimates the basic physical condition of the person in the predetermined basic physical condition estimation time by analyzing the time-series variations relevant to the states of the bioregulation function elements which variations are found by the bioregulation function element judging means, in order of priorities set in advance, and referring to predetermined criteria.

Preferably, the basic physical condition estimating means includes: a first basic physical condition estimating means which estimates that the basic physical condition of the person in the predetermined basic physical condition estimation time is on a predetermined level when the time-series variation of the bioregulation function element having a high priority, out of the time-series variations relevant to the bioregulation function elements which variations are found by the bioregulation function element judging means, satisfies a predetermined criterion; and a second basic physical condition estimating means which, when the estimation is not made by the first basic physical condition estimating means, estimates that the basic physical condition of the person is on any of predetermined levels classified based on a predetermined criterion, by using the time-series variation relevant to another bioregulation function element having a lower priority than the priority of the bioregulation function element used in the first basic physical condition estimating means, wherein the basic physical condition outputting means outputs the level of the basic physical condition of the person estimated by the first basic physical condition estimating means or the second basic physical condition estimating means.

Preferably, by using the time-series variation of the index highly correlated with the autonomic nervous function or the index highly correlated with the bodily and mental fatigue out of the plural bioregulation function elements on which the judgment is made by the bioregulation function element judging means, the first basic physical condition estimating means estimates that the level of the basic physical condition of the person is "good" or "poor" when the time-series variation satisfies a predetermined criterion.

Preferably, the first basic physical condition estimating means estimates that the basic physical condition of the person is "poor" when the time-series variation of the index highly correlated with the autonomic nervous function satisfies a predetermined criterion, and estimates that the basic physical condition of the person is "good" when the time-series variation of the index highly correlated with the bodily and mental fatigue satisfies a predetermined criterion.

Preferably, when the "good" or "poor" estimation is not made by the first basic physical condition estimating means, the second basic physical condition estimating means estimates the level of the basic physical condition of the person as one of "good", "poor", and "intermediate state" from the time-series variation of the index highly correlated with the sensation out of the plural bioregulation function elements on which the judgment is made by the bioregulation function element judging means.

A biological state estimation method of the present invention is a biological state estimation method which analyzes a biosignal measured from a person by a biosignal measurement device and estimates a biological state by using a computer, the method including: a bioregulation function element judging procedure which analyzes the biosignal and calculates a plurality of indexes indicating variations of states of bioregulation function elements, including an index ascribable to fluctuation highly correlated with a brain function, an autonomic nervous function, bodily and mental fatigue, or sensation, every predetermined judgment times set for the respective bioregulation function elements in advance to find time-series variations of the indexes; a basic physical condition estimating procedure which estimates a basic physical condition of the person in a basic physical condition estimation time set longer than the judgment times of the bioregulation function elements in the bioregulation function element judging procedure; and a basic physical condition outputting procedure which outputs a level of the basic physical condition of the person estimated by the basic physical condition estimating means, every time the predetermined basic physical condition estimation time passes, wherein the basic physical condition estimating procedure estimates the basic physical condition of the person in the predetermined basic physical condition estimation time by analyzing the time-series variations relevant to the states of the bioregulation function elements which variations are found by the bioregulation function element judging procedure, in order of priorities set in advance, and referring to predetermined criteria.

Preferably, the basic physical condition estimating procedure of the biological state estimation method includes: a first basic physical condition estimating procedure which estimates that the basic physical condition of the person in the predetermined basic physical condition estimation time is on a predetermined level when the time-series variation of the bioregulation function element having a high priority, out of the time-series variations relevant to the bioregulation function elements which variations are found by the bioregulation function element judging procedure, satisfies a predetermined criterion; and a second basic physical condition estimating procedure which, when the estimation is not made by the first basic physical condition estimating procedure, estimates that the basic physical condition of the person is on any of predetermined levels classified based on a predetermined criterion, by using the time-series variation relevant to another bioregulation function element having a lower priority than the priority of the bioregulation function element used in the first basic physical condition estimating procedure, wherein the basic physical condition outputting procedure outputs the level of the basic physical condition of the person estimated by the first basic physical condition estimating procedure or the second basic physical condition estimating procedure.

A computer program of the present invention is a computer program causing a computer as a biological state estimation device to analyze a biosignal measured from a person by a biosignal measurement device and execute a biological state estimating procedure for estimating a biological state, the computer program causing the computer to execute, as the biological state estimating procedure: a bioregulation function element judging procedure which analyzes the biosignal and calculates a plurality of indexes indicating variations of states of bioregulation function elements, including an index ascribable to fluctuation highly correlated with a brain function, an autonomic nervous function, bodily and mental fatigue, or sensation, every predetermined judgment times set for the respective bioregulation function elements in advance to find time-series variations of the indexes; a basic physical condition estimating procedure which estimates a basic physical condition of the person in a basic physical condition estimation time set longer than the judgment times of the bioregulation function elements in the bioregulation function element judging procedure; and a basic physical condition outputting procedure which outputs a level of the basic physical condition of the person estimated by the basic physical condition estimating procedure, every time the predetermined basic physical condition estimation time passes, wherein the basic physical condition estimating procedure estimates the basic physical condition of the person in the predetermined basic physical condition estimation time by analyzing the time-series variations relevant to the states of the bioregulation function elements which variations are found by the bioregulation function element judging procedure, in order of priorities set in advance, and referring to predetermined criteria.

Preferably, the basic physical condition estimating procedure of the computer program executes: a first basic physical condition estimating procedure which estimates that the basic physical condition of the person in the predetermined basic physical condition estimation time is on a predetermined level when the time-series variation of the bioregulation function element having a high priority, out of the time-series variations relevant to the bioregulation function elements which variations are found by the bioregulation function element judging procedure, satisfies a predetermined criterion; and a second basic physical condition estimating procedure which, when the estimation is not made by the first basic physical condition estimating procedure, estimates that the basic physical condition of the person is on any of predetermined levels classified based on a predetermined criterion, by using the time-series variation relevant to another bioregulation function element having a lower priority than the priority of the bioregulation function element used in the first basic physical condition estimating procedure, wherein the basic physical condition outputting procedure outputs the level of the basic physical condition of the person estimated by the first basic physical condition estimating procedure or the second basic physical condition estimating procedure.

Preferably, by using the time-series variation of the index highly correlated with the autonomic nervous function or the index highly correlated with the bodily and mental fatigue out of the plural bioregulation function elements on which the judgment is made by the bioregulation function element judging means, the first basic physical condition estimating procedure of the computer program estimates that the level of the basic physical condition of the person is "good" or "poor" when the time-series variation satisfies a predetermined criterion.

Preferably, when the "good" or "poor" estimation is not made by the first basic physical condition estimating procedure, the second basic physical condition estimating procedure of the computer program estimates the level of the basic physical condition of the person as one of "good", "poor", and "intermediate state" from the time-series variation of the index highly correlated with the sensation out of the plural bioregulation function elements on which the judgment is made by the bioregulation function element judging procedure.

### Effect of the Invention

According to the present invention, the plural indexes indicating the variations of the states of the bioregulation function elements, including the indexes ascribable to the fluctuation highly correlated with the brain function, the autonomic nervous function, the bodily and mental fatigue, or the sensation are calculated every predetermined judgment times set for the respective bioregulation function elements in advance, their time-series variations are found, the time-series variations are analyzed in order of the priorities, and consequently it is possible to find the physical condition from fluctuation performance which is a basis of the regulation system of a person in the basic physical condition estimation time set longer than the judgment times of the bioregulation function elements. Specifically, in order to estimate the basic physical condition in the predetermined basic physical condition estimation time based on the time-series variations found for the respective plural bioregulation function elements that have antinomy, the priority is statistically found for each fluctuation performance, and by using the indexes of the bioregulation function elements in order of the priorities, the level of the basic physical condition, for example, "good" or "poor", in the basic physical condition estimation time is estimated, and further when the basic physical condition cannot be classified based on the bioregulation function used in this estimation, the level of the basic physical condition corresponding to disturbance of the regulation system, for example, "good", "poor", "intermediate state", or the like is estimated by using the time-series variation relevant to another bioregulation function having a low priority, and output.

Therefore, in the present invention, not only the judgment results of the involved regulation function elements of an organism (bioregulation function elements) are output separately according to the physical condition level, but also the basic physical condition is estimated from the homeostasis function that the fluctuation has, by using the judgment results of the bioregulation function elements, and is output, separately from the judgment results of the bioregulation function elements. This enables a driver, a manager, or the like to grasp the basic physical condition during the past thirty minutes, one hour, or the like at a glance. Accordingly, from the accumulation of this information, the driver or the like himself/herself can decide to take a rest. It is, of course, preferable to co-use a conventional warning based on the judgment results of the individual bioregulation function elements or to give priority to such a warning, but periodically outputting the basic physical condition enables to more appropriately promote a decision to take a rest or other necessary action (warning or the like from the manager).

### Brief Description of Drawings

[FIGs. 1] FIG. 1(a) is an exploded view illustrating an example of a biosignal measurement device, used in one embodiment of the present invention, which measures a back body surface pulse wave, and FIG. 1 (b) is a sectional view illustrating an essential part thereof.
[FIG. 2] FIG. 2 is a diagram schematically illustrating the configuration of a biological state estimation device according to the embodiment of the present invention.
[FIGs. 3] FIG. 3(a) is a chart illustrating an example of frequency gradient time-series waveforms by a zero-cross method and a peak detection method, which are found by a frequency gradient time-series waveform calculating means, and FIG. 3(b) illustrates waveforms resulting from smoothing processing of FIG. 3(a) for finding amplitude fluctuations.
[FIG. 4] FIG. 4 is a chart illustrating an example of time-series waveforms of distribution ratios found by a distribution ratio calculating means.
[FIG. 5] FIG. 5 is a chart illustrating an example of fatigue curves found by a fatigue curve calculating means.
[FIG. 6] FIG. 6 is a chart illustrating an example of a time-series waveform of a homeostasis function level found by a homeostasis function level calculating means.
[FIGs. 7] FIG. 7(a) is a chart illustrating an example of a physical condition map found by a physical condition map calculating means, and FIG. 7(b) is a chart illustrating an example of a sensation map found by a sensation map calculating means.
[FIG. 8] FIG. 8 is a flowchart for explaining steps of estimating a basic physical condition by a basic physical condition estimating means.
[FIGs. 9] FIGs. 9(a), (b) are charts illustrating experiment results of subjects A and B in a given month, in an experiment example 2.
[FIGs. 10] FIGs. 10 are charts illustrating time-series data of physical condition estimation results of the subject B by the biological state estimation device on the 14th day and the 15th day illustrated in FIG. 9(b), and operation start times on operation days before and after these days.
[FIG. 11] FIG. 11 is a distribution chart in which correlations between subjective evaluations by all subjects in the experiment example 2 and ratios of "good" in physical condition estimation by the biological state estimation device are normalized.

### Description of Embodiment

The present invention will be hereinafter described in more detail based on an embodiment of the present invention illustrated in the drawings. A biosignal collected in the present invention is, for example, finger plethysmogram, sound and vibration information collected from the back (hereinafter, "back sound and vibration information"), or the like, and is preferably the back sound and vibration information. As described above, the back sound and vibration information is the sound and vibration information arising from the motions of the heart and the aorta, which is detected from the back of the upper body of a person, and includes information on the systole and diastole of the ventricles, information on the vascular wall elasticity serving as an auxiliary pump for blood circulation, and information on elasticity by blood pressure, and information on reflected waves, that is, it includes information on a back body surface pulse wave (APW) and pseudo heart sound. Further, a signal waveform accompanying heart rate variability includes information on neural activity of the sympathetic nervous system and the parasympathetic nervous system (information on the parasympathetic nervous system activity including compensation for the sympathetic nervous system), while a signal waveform accompanying aortic oscillation includes information on the sympathetic nerve activity and information on the endocrine system, and thus the back sound and vibration information is suitable for the judgment on the bioregulation function elements from different viewpoints.

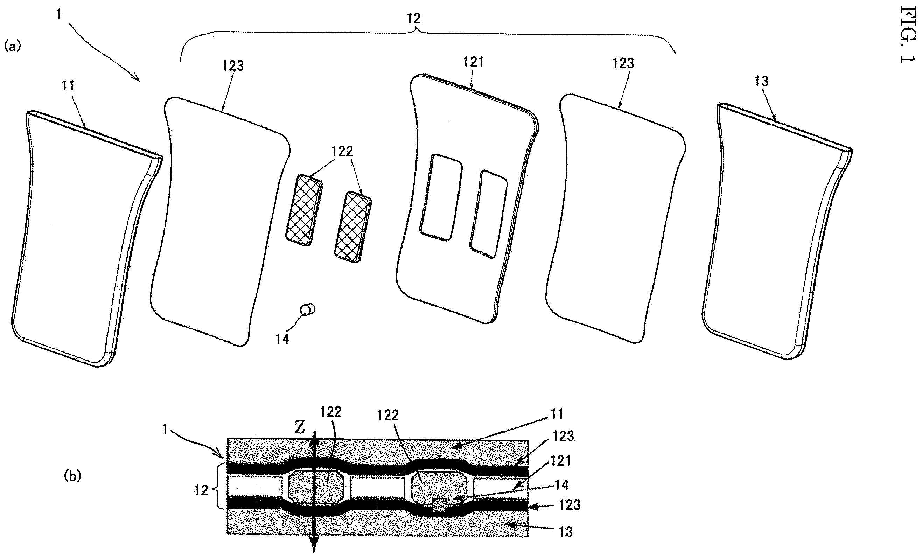

As the biosignal measurement device for collecting the biosignal, a finger plethysmogram meter is usable if the biosignal is finger plethysmogram. If the biosignal is the back sound and vibration information, a pressure sensor is usable, for instance, but preferably, a biosignal measurement device 1 used in a drowsy driving warning device (Sleep Buster (registered trademark)) manufactured by Delta Tooling Co., Ltd. is used. FIGs. 1 illustrate the schematic structure of the biosignal measurement device 1. The biosignal measurement device 1 can be assembled in a driver seat of a vehicle when used and is capable of collecting biosignals without restraining hands or fingers.

The biosignal measurement device 1 will be briefly described. As illustrated in FIGs. 1(a), (b), the biosignal measurement device 1 has a three-layer structure in which a first layer 11, a second layer 12, and a third layer 13 are stacked from the top, and when it is used, the first layer 11 formed of a three-dimensional knitted fabric or the like is located on a human body side being a biosignal detection target. Therefore, a biosignal from the back of the trunk of the human body, in particular, sound and vibration information (back body surface pulse wave (including APW)) of the cardiovascular system including biosound generated in accordance with the vibration of the ventricles, the atrium, and great vessels (direct sound from the trunk or a bioacoustic signal) is propagated first to the first layer 11 being a biosignal input system. The second layer 12 functions as a resonance layer which emphasizes the biosignal, in particular, the sound and vibration of the cardiovascular system propagated from the first layer 11, by means of a resonance phenomenon or a beat phenomenon, and includes a casing 121 formed of a bead foam, three-dimensional knitted fabrics 122 functioning as natural oscillators, and a film 123 generating membrane vibration. In the second layer 12, a microphone sensor 14 is disposed to detect the sound and vibration information. The third layer 13 is stacked opposite the first layer 11 with the second layer 12 therebetween and reduces external sound and vibration input.

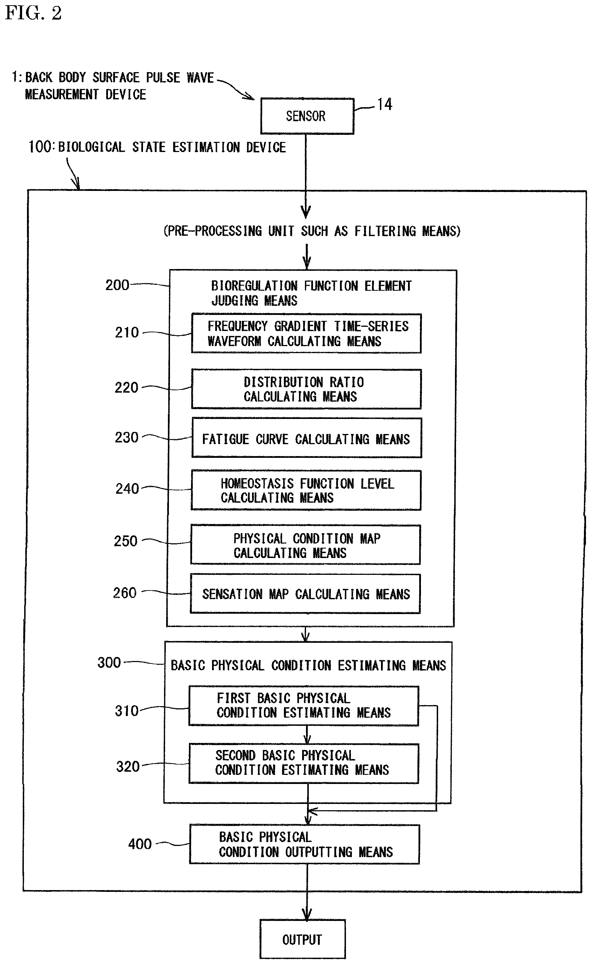

Next, the configuration of the biological state estimation device 100 of this embodiment will be described based on FIG. 2. The biological state estimation device 100 includes a bioregulation function element judging means 200, a basic physical condition estimating means 300, and a basic physical condition outputting means 400. The biological state estimation device 100 is constituted by a computer (including a microcomputer or the like), and in its storage, a computer program causing the computer to execute a bioregulation function element judging procedure, a basic physical condition estimating procedure, and a basic physical condition outputting procedure which execute a biological state estimating procedure and which function as the bioregulation function element judging means 200, the basic physical condition estimating means 300, and the basic physical condition outputting means 400 are set.

The computer program may be stored in a recording medium. By using the recording medium, it is possible to, for example, install the aforesaid program in the aforesaid computer. Here, the recording medium in which the aforesaid program is stored may be a non-transitory recording medium. The non-transitory recording medium is not limited, and examples thereof include recording mediums such as a flexible disk, a hard disk, CD-ROM, MO (magneto-optical disk), DVD-ROM, and a memory card. It is also possible to install the aforesaid program by transmitting it to the aforesaid computer via communication lines.

The bioregulation function element judging means 200, in this embodiment, analyzes the back sound and vibration information which is the biosignal measured by the aforesaid biosignal measurement device 1, and calculates a plurality of kinds of indexes relevant to bioregulation function elements which are to be used in the estimation of a basic physical condition of a person, every predetermined judgment times set for the respective bioregulation function elements in advance to find time-series variations of the respective indexes. Incidentally, the biosignal being an analysis target may be finger plethysmogram or the like, but, in the present invention, is preferably the back sound and vibration information as described above, though the finger plethysmogram is not excluded in the present invention.

The plural kinds of bioregulation function elements on which the judgment is made by the bioregulation function element judging means 200 are not limited, but preferably include at least indexes highly correlated with a brain function and an autonomic nervous function, an index highly correlated with bodily and mental fatigue, and an index highly correlated with sensation. This is because these are indexes indicating how brain wave fluctuation having an influence on a homeostasis function of a person varies or indicating how a bioregulation function represented by a body temperature regulation function works, and the physical condition has a great influence on these regulation functions.

Examples of the indexes highly correlated with the brain function and the autonomic nervous function include a frequency gradient time-series waveform obtained by processing of the collected biosignals, time-series waveforms of distribution ratios of the three signals described in the above Background Art, a fatigue curve, and a time-series variation of the judgment of homeostasis function level. The frequency gradient time-series waveform that is at the base of the regulation operation of the homeostasis function indicates fluctuation, and this fluctuation well regulates the balance of functions having antinomy and is especially highly correlated with the autonomic nervous function of a person. This has been backed up by a statistical method. The time-series waveforms in the respective frequency bands found from the distribution ratios correspond to the kinds of the brain wave (*θ* wave, *α* wave, *β* wave) indirectly involved in a fluctuation rhythm and are highly correlated with the regulation function of the endocrine system in addition to the brain function and the autonomic nervous function of a person. Since the homeostasis is maintained by various regulation systems such as the endocrine system and the autonomic nervous system, variation of its level is deeply correlated also with regulation performance by the fluctuations of the brain, the autonomic nervous system, and the endocrine system.

The bioregulation function element judging means 200 includes the following means as calculating means for finding indexes indicating how the fluctuations of the aforesaid brain function, autonomic nervous function, and endocrine system vary: a frequency gradient time-series waveform calculating means 210 which finds the frequency gradient time-series waveform; a distribution ratio calculating means 220 which finds the distribution ratios; a fatigue curve calculating means 230 which finds the fatigue curve; and a homeostasis function level calculating means 240 which finds the homeostasis function level.

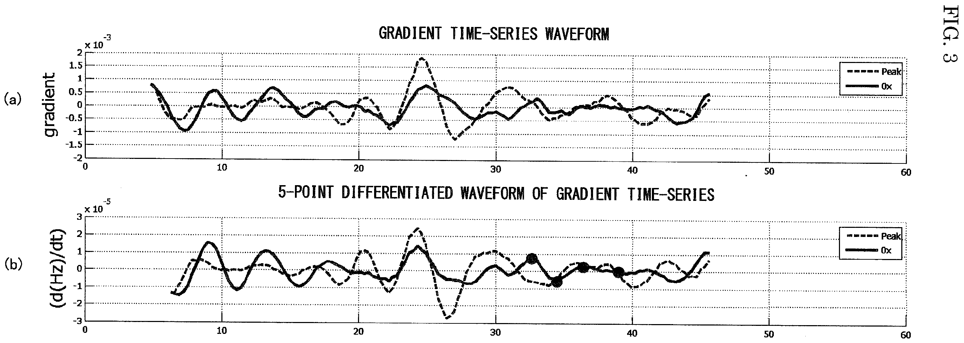

The frequency gradient time-series waveform calculating means 210 finds a time-series waveform of frequency from a time-series waveform of a back body surface pulse wave (APW) of around 1 Hz resulting from filtering of the back sound and vibration information obtained from the sensor 14 of the biosignal measurement device 1, and thereafter finds the frequency gradient time-series waveform (refer to FIGs. 3(a), (b)) by slide calculation of the time-series waveform of the frequency. As the frequency gradient time-series waveform calculating means 210, there are the following two methods as disclosed in the aforesaid Patent Documents 1 and so on: a method (zero-cross method) using switching points from positive to negative (zero-cross points) in the time-series waveform of the back body surface pulse wave (APW); and a method (peak detection method) performing smoothing differentiation of the time-series waveform of the back body surface pulse wave (APW) to find the time-series waveform using peak values (peak points).

In the zero-cross method, after the zero-cross points are found, they are divided in, for example, five-second segments, and a reciprocal of a time interval between the zero-cross points of the time-series waveform included in each five-second period is found as an individual frequency f, and an average value of the individual frequencies f in the five-second period is adopted as a value of a frequency F in the five-second period. Then, the frequencies F obtained every five seconds are plotted in a time-series manner, whereby the time-series waveform of variation of the frequency is found.

In the peak detection method, the aforesaid time-series waveform of the back body surface pulse wave (APW) is subjected to a smoothing differentiation method by, for example, Savitzky and Golath, whereby the peak values are found. Next, the peak values are divided in, for example, five-second segments, a reciprocal of a time interval between the peak values of the time-series waveform included in the five-second period is found as an individual frequency f, and an average value of the individual frequencies f in the five-second period is adopted as a value of a frequency F in the five-second period. Then, the frequencies F obtained every five seconds are plotted in a time-series manner, whereby the time-series waveform of the variation of the frequency is found.

In the time-series waveform of the variation of the frequency found by the zero-cross method or the peak detection method, the frequency gradient time-series waveform calculating means 210 sets time windows each with a predetermined time width (for example, 180 seconds) overlapping with each other by a predetermined time (for example, eighteen seconds), and finds a gradient of the frequency in each of the time windows by a least square method to output the time-series waveform of the gradient. This slide calculation is repeated in sequence, and the time-series variation of the gradient of the frequency of APW is output as the frequency gradient time-series waveform.

The back body surface pulse wave (APW) is a biosignal mainly including the state of control of the heart which is the central nervous system, that is, it is a biosignal including information on the state of sympathetic innervation of arteries and information on the manifestation of the sympathetic nervous system and the parasympathetic nervous system. The frequency gradient time-series waveform found by the zero-cross method (the waveform denoted by "0x" in FIGs. 3(a), (b)) is more correlated with the state of the control of the heart and reflects the manifestation state of the sympathetic nervous system, while the frequency gradient time-series waveform found by the peak detection method found by the peak detection method (the waveform denoted by "Peak" in FIGs. 3(a), (b)) is more correlated with the heart rate variability. Therefore, in order to more clearly grasp the state of the autonomic nervous function, the use of the frequency gradient time-series waveform found by the zero-cross method is preferable.

The activity of the sympathetic nerves has an influence on vascular elasticity and vascular diameter, and an influence of reflected waves from vascular walls is superimposed on a waveform component between pseudo first sound (corresponding to first heart sound) and pseudo second sound (corresponding to second heart sound) of pseudo heart sound information (this is detected from the back and therefore detected as a signal of around 20 Hz from muscles, skin, and so on in a region from the heart to the back surface) included in the sound and vibration information detected from the back of a person. This is a reason why the width between the zero-cross points in the zero-cross method and the width between the peaks in the peak detection method are made different from each other, and in the zero-cross method, the cycle is influenced by the reflected waves. Therefore, by seeing the frequency gradient time-series waveform found by the zero-cross method, it is possible to acquire the information on the sympathetic nerves.

On the other hand, the peak values reflect the information on the heart rate variability as described above, and the heart rate variability is controlled mainly by the parasympathetic nervous system. Therefore, by seeing the peak values, it is possible to acquire the information on the parasympathetic nerves.

It is known that the frequency gradient time-series waveform by the zero-cross method obtained by the frequency gradient time-series waveform calculating means 210 can be regarded as an index of a hypnagogic symptom phenomenon when its amplitude increases in accordance with a temporary increase of the sympathetic nerve activity occurring as resistance against sleepiness at a predetermined timing before sleep and its cycle exhibits a tendency of becoming longer (refer to Patent Document 4). It is also known that, when the waveform indicating the hypnagogic symptom phenomenon exhibits a convergence tendency after appearing and thereafter exhibits a large variation fluctuation with a longer cycle, a point at which the longer-cycle fluctuation is exhibited can be regarded as an index indicating an imminent sleep phenomenon immediately before falling asleep.

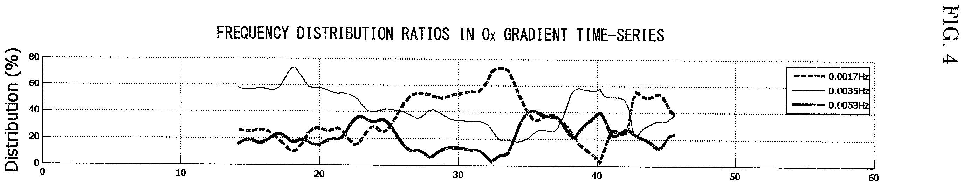

The distribution ratio calculating means 220 first frequency-analyzes the frequency gradient time-series waveform obtained from the frequency gradient time-series waveform calculating means 210 and extracts frequency components belonging to the ULF band to the VLF band, which correspond to the function regulation signal whose frequency is lower than 0.0033 Hz being a frequency at which a fluctuation characteristic of the cardiocirculatory system changes, the fatigue reception signal higher in frequency than the function regulation signal, and the activity regulation signal higher in frequency than the fatigue reception signal. Next, the distribution ratios of these respective frequency components are found in a time-series manner. That is, the ratios of the respective frequency components with the total value of power spectra of the three frequency components being set as 1 are found in a time-series manner as the distribution ratios (refer to FIG. 4).

In this embodiment, as illustrated in FIG. 4, a 0.0017 Hz frequency component is used as the function regulation signal, a 0.0035 Hz frequency component is used as the fatigue reception signal, and a 0.0053 Hz frequency component is used as the activity regulation signal. In atrial fibrillation which is one of heart diseases, the frequency at which the fluctuation characteristic of the cardiocirculatory system switches is said to be 0.0033 Hz, and by finding a fluctuation change near 0.0033 Hz, it is possible to obtain information about the activity of autonomic nerves and homeostasis. Further, it is said that frequency bands near 0.0033 Hz or less and near 0.0053 Hz are relevant mainly to body temperature regulation, and a frequency band of 0.01 to 0.04 Hz are relevant to the control of the autonomic nerves. When the present inventors actually found a frequency gradient time-series waveform used for the calculation of fluctuations of these low frequencies included in the biosignal and frequency-analyzed this, it was confirmed that there are fluctuation of 0.0017 Hz lower than 0.0033 Hz, fluctuation in a frequency band of near 0.0033 Hz around 0.0035 Hz, and in addition to these two, fluctuation in a frequency band around 0.0053 Hz. However, the frequency components of the respective signals can also be adjusted depending on individual difference or the like, and when used, the function regulation signal can be adjusted within a range of less than 0.0033 Hz, preferably within a range of 0.001 to 0.0027 Hz, the fatigue reception signal can be adjusted within a range of 0.002 to 0.0052 Hz, and the activity regulation signal can be adjusted within a range of 0.004 to 0.007 Hz.

As disclosed in Patent Document 2, in the time-series variations of the distribution ratios found by the distribution ratio calculating means 220, a point in time when, for example, the distribution ratio of 0.0017 Hz rapidly decreases and the distribution ratio of 0.0053 Hz exhibits a rapidly increasing change can be regarded as a point in time when the imminent sleep phenomenon appears.

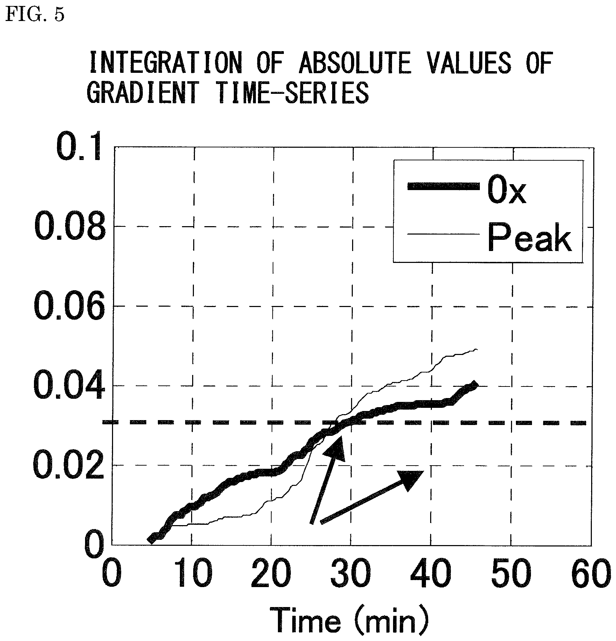

The fatigue curve calculating means 230, which is a means disclosed in Japanese Patent Application Laid-open No. 2009-22610 by the present inventors, is a means which calculates an integrated value by absolute-value processing of the frequency gradient time-series waveform found by the zero-cross method, finds the integrated value as a fatigue degree every predetermined judgment time, plots them in correspondence to the respective times, and finds the fatigue curve illustrated in FIG. 5. Muscle activity is muscular contraction and relaxation, and the fatigue curve which is integration information of the frequency gradient time-series waveform by the zero-cross method reflecting the information on the sympathetic nerves is highly correlated with the muscle activity (refer to Non-patent Document 1). Therefore, in the fatigue curve, a point at which its gradient varies by a predetermined amount or more indicates a singular point, and each singular point indicates a point at which the muscle activity occurs or a point at which a volume of blood flow increases, in accordance with increasing fatigue.

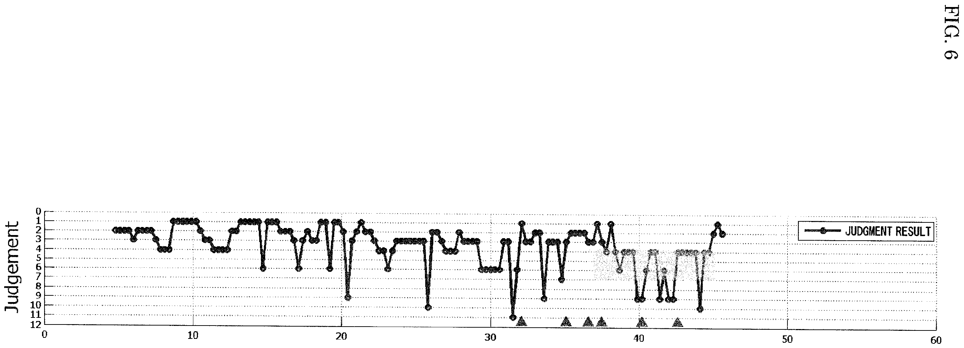

The homeostasis function level calculating means 240, which is based on the art disclosed in Patent Document 3, uses for the judgment, at least one or more of plus/minus of the differentiated waveform of the frequency gradient time-series waveform by the zero-cross method obtained by the frequency gradient time-series waveform calculating means 210, plus/minus of the integrated waveform resulting from the integration of the frequency gradient time-series waveform, the absolute values of the frequency gradient time-series waveforms resulting from the absolute value processing of the frequency gradient time-series waveform found by the zero-cross method and the frequency gradient time-series waveform found by the peak detection method, and so on. Based on the combination of these, to which level the homeostasis function level corresponds is found. For example, the setting can be made such that, by using the frequency gradient and the integrated value, it is judged that the "homeostasis function level is 1" when they are equal to or more than predetermined values, or it is judged that the "homeostasis function level is 4"when the differential value is equal to or less than a predetermined value and out of the two absolute values, "peak is superior". Then, for example, the aforesaid conditions are variously combined and the correlation with the state of a person is found, and cases where the level is judged as 1 to 3 are judged as normal to good states, cases where the level is judged as 4 to 6 are judged as a state requiring caution. Further, cases where it is judged that a hypnagogic symptom or an imminent sleep symptom is occurring are given indexes such as levels 7 to 11 according to the respective states, as a level requiring an immediate warning. In "Sleep Buster" (brand name) manufactured by Delta Tooling Co., Ltd., the setting is made such that the judgment results by the homeostasis function level judging means 240 are displayed as illustrated in FIG. 6, for instance.

As the index highly correlated with bodily and mental fatigue, the physical condition map and the sensation map being indexes disclosed in Patent Document 2 are usable. In these maps, the ways the fluctuations vary are graphed, and they are highly correlated with the bodily and mental fatigue of a person.

Therefore, the bioregulation function element judging means 200 of this embodiment further includes a physical condition map calculating means 250 and a sensation map calculating means 260. The back body surface pulse wave (APW) obtained from the back sound and vibration information obtained from the biosignal measurement device 1 is frequency-analyzed, and an analyzed waveform in a target analysis section is displayed on log-log axes, the analyzed waveform is classified into a low-frequency band, an intermediate-frequency band, and a high-frequency band, the analyzed waveform is scored according to the gradient of the classified analyzed waveform and the whole shape of the analyzed waveform based on a predetermined criterion, and the scores are plotted on the coordinate axes. The physical condition map shows the state of the control of the autonomic nervous system from a viewpoint of the balance between the sympathetic nerves and the parasympathetic nerves, and in the sensation map, the state of a change of heart rate variability is superimposed on the physical condition map.

Specifically, the physical condition map calculating means 250 first finds a regression line in each predetermined cycle area regarding the analyzed waveform resulting from the frequency analysis of the back body surface pulse wave. Next, each of the regression lines found in the respective cycle areas is given an area score based on its gradient, the number of bend points indicating a bifurcation phenomenon is found in each of the whole regression lines based on a discrepancy in a value of the power spectrum density between the regression lines and a gradient difference between the regression lines in the adjacent frequency regions, the shape score based on the number of the bend points is given, and by using at least one of the area score and the shape score, a judgment reference point is found for each of the analyzed waveforms. As the area score, the gradient of each of the regression lines in the respective areas is classified into three, that is, a substantially horizontal state, an upward state, and a downward state, and by using, for example, the score of the substantially horizontal state as a reference, the upward state and the downward state are given higher and lower scores than the reference score respectively. As the shape score, a higher score is given as the number of the bend points is smaller.

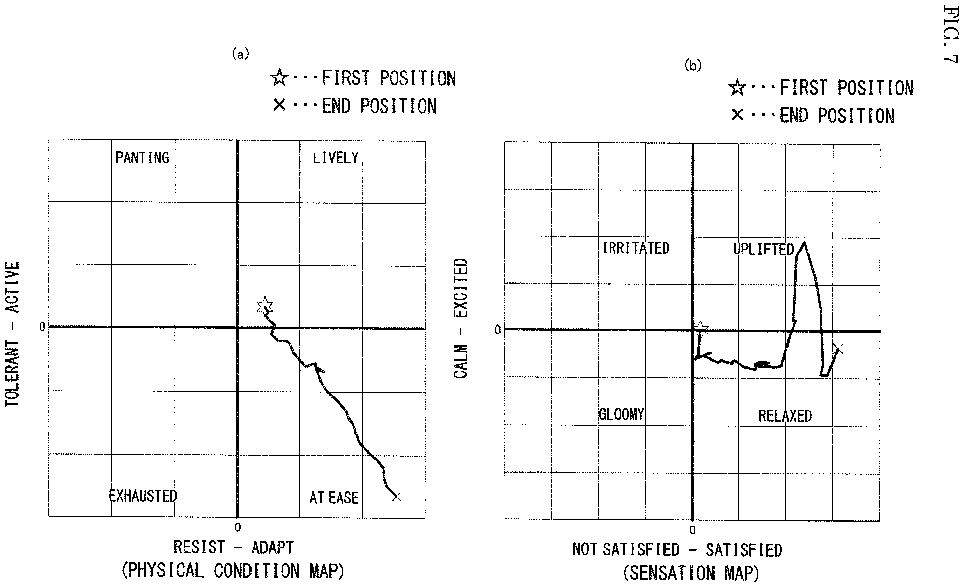

To find the judgment reference point, a first judgment reference point is found using the frequency gradient time-series waveform found by the zero-cross method, and a second judgment reference point is found using the frequency gradient time-series waveform found by the peak detection method. Then, with an index based on the first judgment reference point being taken on one of the axes and an index based on the second judgment reference point being taken on the other axis, coordinate points are plotted, whereby the physical condition map exemplified in FIG. 7(a) is created. In the physical condition map, a case where a coordinate time-series variation line connecting the coordinate points has a variation tendency approximate to a 1/f gradient is judged as comfortable, and a case where it is judged that this coordinate time-series variation line varies in an up and down direction is judged as uncomfortable. In FIG. 7(a), the plural coordinate points are connected without any coordinate point being plotted at the origin of coordinates, but in order to see a variation tendency between two points different in terms of time, the first one is plotted at the origin of coordinates, and a case where the second one is plotted in the fourth quadrant is judged as "good" regarding this bioregulation function element, which facilitates the determination.

The sensation map calculating means 260 is a means which performs moving calculation in which a frequency average value is found in each of predetermined time windows set with a predetermined overlap time in the heart rate variability-relevant frequency time-series waveform found by the peak detection method, finds a time-series variation of the frequency average value obtained in each of the time windows as a frequency variation time-series waveform, and with an index corresponding to a functional point found from the frequency time-series waveform found by the zero-cross method being taken on one of the axes and with an index corresponding to a variation amount in a predetermined time width of the aforesaid frequency variation time-series waveform found by the peak detection method being taken on the other axis, finds a coordinate time-series variation found from the functional point and the variation amount. FIG. 7(b) is an example of the sensation map found in this way. In FIG. 7(b), a plurality of coordinate points are connected without any coordinate point being plotted at the origin of coordinates, but in a case where a variation tendency between two points different in terms of time is to be seen, the first one is plotted at the origin of coordinates and the second one is plotted, which facilitates the determination of a separation distance and a separation direction of the both.

The functional point is found between the judgment reference points of the analyzed waveform in two former and latter time ranges being comparison targets, by the following expression:
functional point = judgment reference point of latter time range + (judgment reference point of latter time range - judgment reference point of former time range) x n, (where n is a correction coefficient).

As for the index highly correlated with the sensation, the judgment can be made by using appearance frequencies of the indexes of the levels that can be regarded as normal to good (in the above example, levels 1 to 3) and the indexes of the levels requiring caution (in the above example, levels 4 to 6), which are judged using the frequency gradient and the integrated value, in the time-series variation of the homeostasis function level found by the aforesaid homeostasis function level calculating means 240. The homeostasis function level is highly correlated with the state of the autonomic nervous function as described above, but when sympathetic nerve compensation is manifested in response to fatigue, tiredness is sometimes felt and is not sometimes felt depending on the physical condition, basic physical strength, or motivation. Therefore, the sympathetic compensation and the basic physical condition with respect to fatigue are highly correlated with sensation with which the fatigue is perceived as fatigue. Note that the sensation mentioned here refers to sensation similar to a sense of loss accompanied by a lassitude feeling or a low conscious state.

The basic physical condition estimating means 300 is a means which estimates a basic physical condition (basic physical condition) of an analysis target person by referring to a predetermined criterion, from time-series variations relevant to fluctuation performances of the bioregulation function elements which variations are found in the aforesaid bioregulation function element judging means 200. The bioregulation function element judging means 200 is set to find the plural kinds of the time-series variations relevant to the fluctuation performances of the bioregulation function elements as described above, and the plural kinds of the found time-series variations are obtained every predetermined judgment times respectively. For example, it takes several minutes for the frequency gradient time-series waveform calculating means 210 to output the first calculation result after obtaining data from the biosignal measurement device 1, but thereafter the calculation result is obtained every eighteen seconds, for instance, and consequently, the time-series variation is found. It takes several minutes to output the first calculation results of the distribution ratios obtained by the distribution ratio calculating means 220, the fatigue degree obtained by the fatigue curve calculating means 230, and the homeostasis function level obtained by the homeostasis function level calculating means 240, and thereafter the calculation results are obtained every eighteen seconds, for instance, whereby the respective time-series variations are obtained. It takes twenty to thirty minutes to first output the calculation results found by the physical condition map calculating means 250 and the sensation map calculating means 260 respectively, but the second results are obtained about ten several minutes later, and the third and subsequent results are obtained every several minutes. On the other hand, the basic physical condition estimating means 300 estimates the basic physical condition in a longer time (basic physical condition estimation time) than the judgment times for the respective bioregulation function elements.

For example, in order to enable a driver to grasp his/her physical condition, it is, of course, possible to display the judgment results of the fluctuation performances of the respective bioregulation function elements individually on a monitor, but in this case, the judgment is likely to vary depending on analysis capability and judgment capability of the driver who analyzes the data. Therefore, it is preferable to estimate the basic physical condition by statistically processing the combination of the data in consideration of their contribution ratios as required after absorbing the variation by mechanical judgment. That is, this is a method to estimate current basic control capability (physical condition) of an organism by statistically combining the data which are output every relatively short time. The judgment results of the bioregulation function elements are suitable for use in grasping a driver's latest psychosomatic state which changes from time to time, but even if the judgment results are successively output while the driver is engaged in work of driving, it is sometimes difficult for the driver himself/herself to analyze the states of the judgment results to realize the basic physical condition. Therefore, the basic physical condition estimating means 300 is configured to estimate the basic physical condition in the basic physical condition estimation time set longer. The basic physical condition estimation time can be set to any period such as fifteen minutes, thirty minutes, or sixty minutes, but too high a frequency and too many state changes result in a difficulty for the driver to grasp his/her physical condition. Accordingly, the basic physical condition estimation time is preferably about twenty to forty minutes.

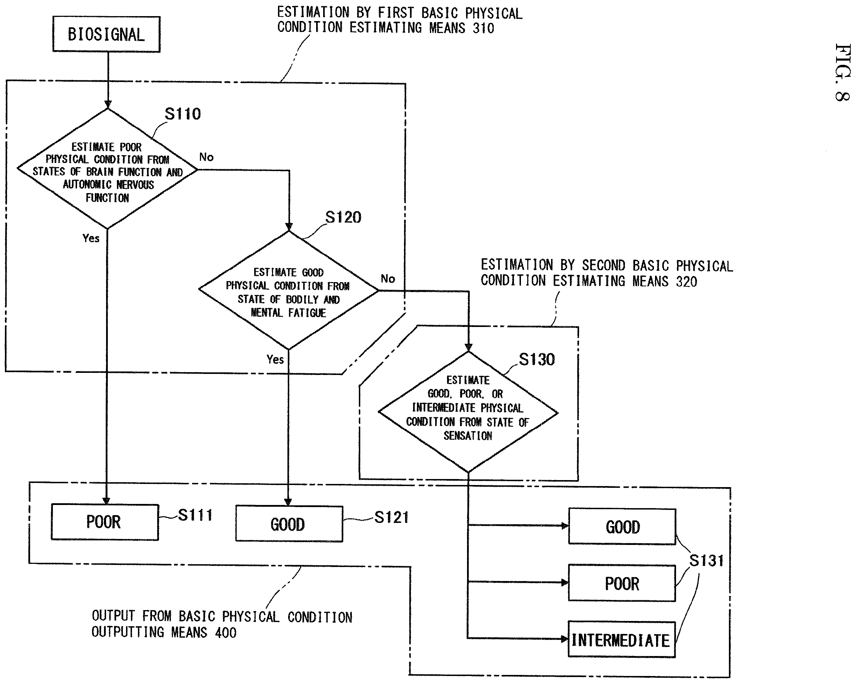

The basic physical condition estimating means 300 judges the basic physical condition using the judgment results of the bioregulation function elements. Specifically, the judgment result of the bioregulation function element more reflecting the physical condition in the basic physical condition estimation time set to a predetermined long time is used with a higher priority. Therefore, in this embodiment, priorities in using the judgment results of the bioregulation function elements are set in the basic physical condition estimating procedure which is a computer program constituting the basic physical condition estimating means 300. In more detail, the basic physical condition estimating means 300 includes a first basic physical condition estimating means 310 and a second basic physical condition estimating means 320 as illustrated in FIG. 2, and is set to first estimate the basic physical condition in the first basic physical condition estimating means 310 (S110, S120), and next estimate the basic physical condition in the second physical condition estimating means 320 (S130) as illustrated in the flowchart in FIG. 8.

The first basic physical condition estimating means 310 finds whether or not the time-series variation of the bioregulation function element having a higher priority, out of the bioregulation function elements for which the priorities are set, satisfies a predetermined criterion, and when it satisfies a predetermined level, estimates the physical condition as the predetermined level. When this time-series variation does not become a judgment target in the first basic physical condition estimating means, that is, when it does not satisfy the predetermined criterion in the first basic physical condition estimating means 310, the second basic physical condition estimating means 320 estimates the basic physical condition based on a predetermined criterion, by using the time-series variation relevant to another bioregulation function element having a lower priority than that of the bioregulation function element used in the first basic physical condition estimating means 310. Therefore, since the second basic physical condition estimating means 320 is a final means for estimating the basic physical condition, the second basic physical condition estimating means 320 is set so as to classify the judgment target data into any of the basic physical conditions without fail.

The priorities of the bioregulation function elements obtained by the bioregulation function element judging means 200, which are estimation targets in the basic physical condition estimating means 300, are preferably decided based on statistical analysis of many cases as in later-described experiment examples, and when necessary, in consideration of their contribution ratios. This enables more improved estimation accuracy. In this embodiment, based on the statistical processing in the later-described experiment examples, the priority of the time-series variation of the index highly correlated with the autonomic nervous function or the index highly correlated with the bodily and mental fatigue is set high, and the priority of the time-series variation of the index highly correlated with the sensation is set next highest. Therefore, the first basic physical condition estimating means 310 is set to judge whether or not the time-series variation of the index highly correlated with the autonomic nervous function satisfies a predetermined condition, and whether or not the time-series variation of the index highly correlated with the bodily and mental fatigue satisfies a predetermined condition. Further, the first basic physical condition estimating means 310 is set to estimate the basic physical condition as "poor" when the time-series variation of the index highly correlated with the autonomic nervous function satisfies the predetermined condition, and estimate the basic physical condition as "good" when the time-series variation of the index highly correlated with the bodily and mental fatigue satisfies the predetermined condition.

The aforesaid frequency gradient time-series waveform, distribution ratio, fatigue curve (fatigue degree), and homeostasis function level which are the indexes relevant to the homeostasis function based on the fluctuations of the brain function, the autonomic nervous function, or the regulation function of the endocrine system are indexes based on which symptoms resulting from fatigue accumulation, such as a hypnagogic symptom, imminent sleep, and a low consciousness traveling state, can be easily found. Further, regulation systems such as the endocrine system governing the fluctuations of the homeostasis functions regulated by the brain function differ depending on a difference in their frequency band, and the distribution ratios out of the above-listed ones are indexes well reflecting a rapid change, attenuation, and intensification of these regulation systems. Therefore, by using these, the basic physical condition is estimated as "poor" when a situation requiring caution or warning during work such as driving prominently appears in the basic physical condition estimation time or it appears at a predetermined frequency or more. The physical condition map and the sensation map which are the indexes highly correlated with the bodily and mental fatigue facilitate prominently finding an index indicating that an excellent condition or comfortableness is felt. Therefore, under the condition where this index indicates an excellent condition or comfortableness, the basic physical condition is estimated as "good". In the estimation of the basic physical condition in the first basic physical condition estimating means 310, which one of the index highly correlated with the brain function, the autonomic nervous function, and the regulation function of the endocrine system and the index highly correlated with the bodily and mental fatigue should be used with a higher priority is not limited, but since a change of the bodily and mental fatigue is also basically correlated with the autonomic nerves, it is preferable to execute the estimation using the index highly correlated with the brain function, the autonomic nervous function, or the like, and next execute the estimation using the index highly correlated with the bodily and mental fatigue, as in this embodiment.

In this embodiment, there are the following four indexes highly correlated with the brain function, the autonomic nervous function, and a hormone secretion regulation function of the endocrine system: the frequency gradient time-series waveform, the distribution ratio, the fatigue curve (fatigue degree), and the homeostasis function level as described above. The setting can be made such that, when it is detected in one of these that a symptom such as a hypnagogic symptom is on a predetermined level or more, the basic physical condition is estimated as "poor", but estimating the basic physical condition as "poor" when a predetermined symptom is detected in a plurality of indexes is preferable in order to enhance reliability.

Therefore, the first basic physical condition estimating means 310 of this embodiment is set to estimate the physical condition as "poor" when three or more indexes out of the frequency gradient time-series waveform found by the frequency gradient time-series waveform calculating means 210, the time-series waveform of the distribution ratio found by the distribution ratio calculating means 210, the fatigue curve (time-series waveform of the fatigue degree) found by the fatigue curve calculating means 230, and the homeostasis function level found by the homeostasis function level calculating means 240 satisfy the predetermined criteria (when the judgment result at S110 in FIG. 8 "Yes").

The predetermined criteria for the "poor" estimation in this embodiment are set as follows.

### (a) The index found from the frequency gradient time-series waveform calculating means 210

A case where the comparison of amplitude changes in the frequency gradient time-series waveform found by the zero-cross method shows that there occurs a convergence place in which the amplitude changes to less than 90 to 60% of an immediately preceding amplitude continuously a plurality of times (typically, set within a range of twice to four times) (an index based on which it is inferred that the sympathetic nerve activity lowers and the subject has got into a state where he/she cannot resist sleepiness)

### (b) The indexes found from the distribution ratio calculating means 220

A case where, in the time-series variations of the distribution ratios in the frequency gradient time-series waveform found by the zero-cross method, the distribution ratio of 0.0017 Hz rapidly decreases (typically, a decrease rate is set to 15% or more) within a predetermined time range (typically, the range is set to 60 to 120 seconds) and during this period, the distribution ratio of 0.0053 Hz rapidly increases (typically, an increase rate is set to 15% or more) (an index for the estimation of the appearance of the hypnagogic symptom)

### (c) The indexes found from the fatigue curve calculating means 230

A case where, in a predetermined time (typically, set within a range of three to ten minutes), there is one place or more at which the gradient of the fatigue curve using the peak detection method (the time-series waveform of the integration of the absolute values of the frequency gradient time-series waveform using the peak detection method) changes to a larger gradient than the gradient of the fatigue curve using the zero-cross method (the time-series waveform of the integration of the absolute values of the frequency gradient time-series waveform using the zero-cross method), and after a predetermined time elapses, the fatigue curve using the peak detection method reaches a predetermined value or more (an index for the estimation that the parasympathetic nerve activity is extremely predominant)

### (d) The index found from the homeostasis function level calculating means 240

When, out of the homeostasis function levels obtained every eighteen seconds, the levels lower than the normal level, in the above example, the caution judgment being the levels 4 to 6, appear several times to ten several times or more (an index appearing when the state in which the parasympathetic nerve activity is predominant is estimated), or when the levels requiring the warning, in the above example, the warning judgment being the levels 7 to 11, appear several times or more (an index appearing when a sudden increase, an extreme decrease, or the like of the sympathetic nerve activity is estimated)

Further, the first basic physical condition estimating means 310 of this embodiment estimates whether or not data not estimated as "poor" in three or more of the aforesaid indexes (a) to (d) (data on which "No" judgment is made at S110 in FIG. 8) corresponds to "good" meaning that the predetermined criterion is satisfied, by using the indexes of the physical condition map 250 and the sensation map 260 (S120 in FIG. 8).

### (e) The indexes based on which the basic physical condition is judged as "good"

The criterion corresponding to the "good" basic physical condition is set in this embodiment such that the basic physical condition is estimated as "good" ("Yes" at S120 in FIG. 8) when, in the time-series variation found by the physical condition map calculating means 250, with a point at which an immediately preceding calculation result is output (as described above, the first one and the second one are output after the predetermined time elapses, but the third and subsequent ones are output every several minutes) being plotted at the origin of coordinates, the next point is plotted in the fourth quadrant, and in the time-series variation found by the sensation map calculating means 260, with an immediately preceding point being similarly plotted at the origin of coordinates, the next point is plotted a predetermined distant more apart in an X-axis direction.

The judgment criteria (a) to (d) for the "poor" estimation of the basic physical condition and the judgment criterion (e) for the "good" estimation of the basic physical condition are based on later-described statistical analysis of many cases, but this is not restrictive. For example, data of each individual may be accumulated and the conditions may be statistically set for each individual.

The second basic physical condition estimating means 320 is executed when estimation target data does not satisfy any of the "poor" and "good" criteria in the first basic physical condition estimating means 310 (when the judgment results at both S110 and S120 in FIG. 8 are "No"). The second basic physical condition estimating means 320 compares the appearance frequencies of levels near the boundary between the indexes corresponding to the levels indicating that the sympathetic nerve activity is predominant and the physical condition can be said as normal to good (levels 1 to 3 in the above example) and the indexes corresponding to the levels indicating that the parasympathetic nerve activity is predominant and caution is required (levels 4 to 6 in the above example), in the time-series variation of the homeostasis function level found by the homeostasis function level calculating means 240. However, a level difference by one stage indicates only a small state difference, and therefore, the comparison is preferably made between levels different by two stages or more. In this embodiment, a ratio of the appearance frequencies of the index corresponding to the level 2 being the middle index out of the three-stage levels that can be said as normal to good and the index corresponding to the level 4 requiring the start of caution are compared. Basically, in a case where the appearance frequency of the level 2 indicating the sympathetic nerve activity predominant state and thus a good state is high and the appearance frequency of the level 4 indicating the parasympathetic nerve activity predominant state and thus the caution state is low, the basic physical condition can be estimated as "good", and in a case where the relation of the appearance frequencies is reversed, the basic physical condition can be estimated as "poor", but since the analysis target data of the second basic physical condition estimating means 320 are those not estimated clearly as "good" or "poor" in the first basic physical condition estimating means 310, there may be data that are difficult to be classified into any of these. Therefore, in the present invention, in the later-described test examples, many cases are analyzed, and criteria for classifying these into "good", "poor", and "intermediate state" are set using a Bayes estimation method. Details will be described later.

In this embodiment, when the aforesaid condition of the first basic physical condition estimating means 310 is satisfied (when, in FIG. 8, the judgment result at S110 is "Yes" or the judgment result at S120 is "Yes"), the basic physical condition outputting means 400 outputs an estimation result indicating that the level of the basic physical condition is "poor" (S111 in FIG. 8) or "good" (S121 in FIG. 8). When the condition in the first basic physical condition estimating means 310 is not satisfied (when, in FIG. 8, the judgment result at S110 is "No" and in addition, the judgment result at S120 is "No"), the basic physical condition outputting means 400 outputs an estimation result of the second basic physical condition estimating means 320, namely, "good", "poor", or "intermediate state" (S131 in FIG. 8). The basic physical condition outputting means 400 outputs "good", "poor", or "intermediate state" being the estimation result of the basic physical condition through a medium recognizable by a person. For example, when the biosignal measurement device 1 and the biological state estimation device 100 of this embodiment are mounted in an automobile to detect the state of a driver, the estimation result is displayed on an in-vehicle monitor every time the basic physical condition estimation time passes. A display method may be letter, pattern, or the like. As the pattern, for example, a "fair" weather symbol is usable in the case of "good", a "rainy" weather symbol is used in the case of "poor", and a "cloudy" weather symbol is usable in the case of "intermediate state", for easy visual recognition by a driver. Besides, a plurality of kinds of characters may be used for the display, or a combination of these may be displayed. Further, sound can be output through an in-vehicle speaker.

According to this embodiment, when a state of, for example, a driver is analyzed, the basic physical condition of the driver can be estimated and output every predetermined physical condition estimation time, in addition to a change of each of the bioregulation function elements in each short time. Therefore, it is easy for the driver himself/herself to roughly realize in what state he/she has been driving for, for example, the past thirty minutes, and if, for example, the basic physical condition is continuously estimated as "poor", it is easy to immediately promote a decision to take a rest or the like. Further, by connecting the in-vehicle biological state estimation device 100 and a computer on a manager side via a communication means in advance, it is possible to grasp in real time not only the individual bioregulation function elements of the driver but also his/her basic physical condition, which will be of help for the determination by the manager side.

### (Experiment example 1)

Next, experiment results regarding the setting of the estimation criteria of the first basic physical condition estimating means 310 and the second basic physical condition estimating means 320 included in the basic physical condition estimating means 300 will be described.

### (Experiment method)

In an automobile, the biosignal measurement device 1 as the biosignal measurement device was mounted on a seat back of a driver seat and the biological state estimation device 100 being a computer receiving data of the back sound and vibration information measured by the biosignal measurement device 1 was mounted. A subject was seated in the automobile to drive. As the biological state estimation device 100, "Sleep Buster" (brand name) manufactured by Delta Tooling Co., Ltd. was used.

The subject was a healthy Japanese male in his sixties and was made to continuously drive for about forty minutes a plurality of times on different days. After finishing the driving, the subject was requested to self-declare his physical condition in the driving. The self-declaration was made in five stages of "good", "somewhat good", "normal", "somewhat poor", and "poor". Ambiguous cases such as "somewhat good", "normal", and "somewhat poor" in the self-declaration were excluded, and traveling data of 55 cases where the clear "good" and "poor" judgment was made by the subject were compared with the estimation results of the basic physical condition obtained by the method of this embodiment.

### I. Estimation of the basic physical condition by the first basic physical condition estimating means 310

### (1) Study on whether or not the index obtained from the frequency gradient time-series waveform calculating means 210 satisfies the aforesaid condition (a)

The condition (a) was set to a case where a convergence place in which the amplitude changed to less than 90% of an immediately preceding amplitude continuously twice occurred once or more, and a chi-squared test of the comparison with the subjectivity by the subject was conducted. The following table shows a 2 x 2 cross-table, in which p = 0.24 and a correct answer ratio was 67%.

**[Table 1]**

| | | Condition (a) satisfied or not | |
|---|---|---|---|
| | | Not satisfied | Satisfied |
| Subjective evaluation by subject | Good | 32 | 6 |
| | Poor | 12 | 5 |

### (2) Study on whether or not the indexes found from the distribution ratio calculating means 220 satisfy the aforesaid condition (b)

The condition (b) was set to a case where the distribution ratio of 0.0017 Hz decreased by 20% or more in 90 seconds, and during this period, the distribution ratio of 0.0053 Hz increased by 20% or more, and a chi-squared test of the comparison with the subjectivity by the subject was conducted. The following table shows a 2 x 2 cross-table, in which p = 0.11 and a correct answer ratio was 69%.

**[Table 2]**

| | | Condition (b) satisfied or not | |
|---|---|---|---|
| | | Not satisfied | Satisfied |
| Subjective evaluation by subject | Good | 32 | 6 |
| | Poor | 11 | 6 |

### (3) Study on whether or not the indexes obtained from the fatigue curve calculating means 230 satisfy the aforesaid condition (c)

The condition (c) was set to a case where, in a six-minute range, there was one place or more at which the gradient of the fatigue curve using the peak detection method changed to 1.5 times or more the gradient of the fatigue curve using by the zero-cross method, and the fatigue curve using the peak detection method became larger than 0.03 after thirty minutes passed, and a chi-squared test of the comparison with the subjectivity by the subject was conducted. The following is a 2 x 2 cross-table, in which p = 0.19 and a correct answer ratio was 64%.

**[Table 3]**

| | | Condition (c) satisfied or not | |
|---|---|---|---|
| | | Not satisfied | Satisfied |
| Subjective evaluation by subject | Good | 27 | 11 |
| | Poor | 9 | 8 |

### (4) Study on whether or not the index obtained from the homeostasis function level calculating means 240 satisfies the aforesaid condition (d)

The condition (d) was set to a case where the level 4 to 6 judgment occurred ten times or more or the level 7 to 11 judgment occurred seven times or more in six minutes in the above-described example, and a chi-squared test of the comparison with the subjectivity by the subject was conducted. The following is a 2 x 2 cross-table, in which p = 0.018 and a correct answer ratio was 71%.

**[Table 4]**

| | | Condition (d) satisfied or not | |
|---|---|---|---|
| | | Not satisfied | Satisfied |
| Subjective evaluation by subject | Good | 30 | 8 |
| | Poor | 8 | 9 |

In the following table, the numbers of data satisfying the aforesaid conditions (a) to (d), out of the traveling data of the 55 cases, are summarized.

**[Table 5]**

| | | Number of traveling data satisfying conditions (a) to (d) | | | | |
|---|---|---|---|---|---|---|
| | | 0 | 1 | 2 | 3 (Poor) | 4 (Poor) |
| Subjective evaluation by subject | Good | 12 | 21 | 5 | 0 | 0 |
| | Poor | 3 | 7 | 0 | 7 | 0 |

As is apparent from Table 5, seven cases in which three or more of the conditions (a) to (d) are satisfied are also all evaluated as "poor" in the subjectivity by the subject. Therefore, the first basic physical condition estimating means 310 is preferably set to make the "poor" estimation when three or more kinds of the indexes, out of the frequency gradient time-series waveform found from the frequency gradient time-series waveform calculating means 210, the time-series waveforms of the distribution ratios obtained from the distribution ratio calculating means 210, the fatigue curves (the time-series waveforms of the fatigue degree) obtained from the fatigue curve calculating means 230, and the homeostasis function level obtained from the homeostasis function level calculating means 240, satisfy the aforesaid conditions (a) to (d) respectively.

### (5) Study on whether or not the aforesaid condition (e) for the "good" basic physical condition judgment is satisfied

Regarding all the traveling data of the 55 cases, studies were made on whether or not a coordinate point of the second judgment result when the first judgment result from the start of the measurement was plotted at the origin of coordinates was plotted in the fourth quadrant by the physical condition map calculating means 250. Similarly, regarding all the traveling data of the 55 cases, studies were made on whether or not the coordinate point of the second judgment result when the first judgment result from the start of the measurement was plotted at the origin of coordinates was plotted at a position apart by a predetermined number of calibrations or more (in this experiment example, the number of calibrations was set to five or more since the separation distance in many cases (23 cases) out of 38 cases with the "good" subjective evaluation was five calibrations or more) in the positive direction of the X axis by the sensation map calculating means 260. When the calculation results of the physical condition map calculating means 250 and the sensation map calculating means 260 both satisfy the aforesaid conditions, the estimation result of the first basic physical condition estimating means 310 is the "good" physical condition, and the result of a chi-squared test of the comparison with the subjectivity by the subject is as in the following table. p = 0.095 and a correct answer ratio was 56%.

**[Table 6]**

| | | Condition (e) satisfied or not | |
|---|---|---|---|
| | | Satisfied (good) | Not satisfied |
| Subjective evaluation by subject | Good | 18 | 20 |
| | Poor | 4 | 13 |

In the above study in Table 6, the traveling data of all the 55 cases are used, and as illustrated in the flowchart in FIG. 8, the first basic physical condition estimating means 310 first estimates the basic physical condition as "poor" when three or more of the aforesaid conditions (a) to (d) are satisfied (SI10), and regarding cases where the "poor" estimation is not made, it determines whether or not they correspond to "good" by using the physical condition map and the sensation map (S120). As a result of comparison and study regarding 48 cases except the seven cases satisfying three or more of the aforesaid conditions (a) to (d), the following result was obtained. As is seen from this table, p = 0.032 and a correct answer ratio was 56% when the seven cases were excluded. p < 0.05, and it is preferable to make the "good" estimation when the "poor" estimation condition is not satisfied.

**[Table 7]**

| | | Condition (e) satisfied or not | |
|---|---|---|---|
| | | Satisfied (good) | Not satisfied |
| Subjective evaluation by subject | Good | 18 | 20 |
| | Poor | 1 | 9 |

### II. Estimation of the basic physical condition by the second basic physical condition estimating means 320

Appearance ratios of the level 2 indicating the sympathetic nerve activity predominant state and the level 4 indicating the parasympathetic nerve activity predominant state, out of the homeostasis function levels found by the homeostasis function level calculating means 240 of the biological state estimation device 100, were found, with the total appearance number of the levels 1 to 6 being set to 1. These were found in all the 55 cases, and the following probability table was created using Bayes estimation.

**[Table 8]**

| Homeostasis function level | | Subjective evaluation by subject | | Probability by Bayes estimation | | Estimated basic physical condition |
|---|---|---|---|---|---|---|
| Ratio of level 2 | Ratio of level 4 | Good | Poor | Probability of good | Probability of poor | |
| 0.35 or less | 0.05 or less | 4 | 0 | 1 | 0 | Good |
| 0.35 or less | Over 0.05 | 9 | 8 | 0.33 | 0.67 | Poor |
| Over 0.35 | 0.05 or less | 14 | 2 | 0.76 | 0.24 | Good |
| Over 0.35 | Over 0.05 | 11 | 7 | 0.41 | 0.59 | Intermediate |

The basic physical condition estimated by the second basic physical condition estimating means 230 using the probability table in Table 8 is as follows.

**[Table 9]**

| | | Basic physical condition estimated by second basic physical condition estimating means | | |
|---|---|---|---|---|
| | | Good | Intermediate | Poor |
| Subjective evaluation by subject | Good | 18 | 11 | 9 |
| | Poor | 2 | 7 | 8 |

In the aforesaid studies in Table 8 and Table 9, the traveling data of all the 55 cases were used for the creation of the probability table, and as illustrated in the flowchart in FIG. 8, the second basic physical condition estimating means 320 estimates the physical condition when the basic physical condition is not estimated by the first basic physical condition estimating means 310. The following table shows results of the comparison and study regarding 29 cases excluding 26 cases regarding which the first basic physical condition estimating means 31 makes the "good" or "poor" estimation.

**[Table 10]**

| | | Basic physical condition estimated by second basic physical condition estimating means | | |
|---|---|---|---|---|
| | | Good | Intermediate | Poor |
| Subjective evaluation by subject | Good | 10 | 8 | 2 |
| | Poor | 1 | 3 | 5 |

In the biological state estimation device 100 of this embodiment, as illustrated in the flowchart in FIG. 8, regarding the estimation target data, the first basic physical condition estimating means 310 first estimates that the physical condition is "poor" (S110), and when the estimation result is not "poor", estimates that the physical condition is "good" (S120), and when the estimation result is neither "poor" nor "good", the second basic physical condition estimating means estimates that the physical condition is "good", "poor", or "intermediate state" (indicated as "intermediate" in the above table) based on the probability table by the Bayes estimation illustrated in Table 8 (S130), but in this experiment example, the 55 cases clearly evaluated as "good" or "poor" by the subject are used.

Therefore, in these 55 cases, the data finally estimated as "intermediate" in the biological state estimation device 100 do not agree with the subjective evaluation of the subject. Therefore, the data estimated as "intermediate" (eleven cases) are excluded. Then, regarding data estimated as "poor" (poor: 7 cases) in the first basic physical condition estimating means 310, data estimated as "good" (good: 19 cases) out of data excluding the data estimated as "poor" by the first basic physical condition estimating means 310, and data estimated as "poor" and "good" in the second basic condition estimating means 320 (poor: 7 cases, good: 11 cases) out of data excluding the data estimated as "poor" and "good" in the first basic physical condition estimating means 310, that is, regarding data not overlapping, the estimation results finally output by the basic physical condition outputting means 400 are compared with the subjective evaluation by the subject. The following table shows the summary of the comparison.

**[Table 11]**

| | | Estimation result of basic physical condition | |
|---|---|---|---|
| | | Good | Poor |
| Subjective evaluation by subject | Good | 28 | 2 |
| | Poor | 2 | 12 |

According to Table 11, p = 1.58 x 10⁻⁷, and a correct answer ratio is 91%, which shows that the estimation result of the basic physical condition by the biological state estimation device 10 of this embodiment agrees with the subjective evaluation by the subject with a high probability, and the estimation result of the basic physical condition of this embodiment is highly reliable.

Incidentally, the "intermediate" estimation on the data of the eleven cases by the second basic physical condition estimating means 320 among the 55 cases excluding the data estimated as "good" or "poor" in the first basic physical condition estimating means 310 do not agree with the subjective evaluation by the subject, a reason for which is thought to be that the subjective evaluation is influenced by basic physical strength and trend of thought of the subject. That is, the reason for this may be that the subject of this experiment example tends to think positively and has physical strength, and in many of the eleven cases, he declared that the physical condition was "good" as the subjectivity evaluation.

### (Experiment example 2)

Next, Sleep Buster (registered trademark) manufactured Delta Tooling Co., Ltd. was mounted as the biosignal measurement device 1 on a driver seat of a truck of a transport company to collect biosignals of professional drivers, and the biological state estimation device 100 in which the estimation criteria described in the experiment example 1 were set analyzed the collected biosignals according to the flowchart illustrated in FIG. 8 to estimate the physical conditions of the subjects in three stages of "good", "intermediate", and "poor" every thirty minutes. The professional drivers being the subjects were seven males in their twenties to fifties, and the analysis was conducted on totally 71 times of operations by all the subjects. Further, after finishing the operations, the subjects were requested to declare their subjective physical conditions on the days of the operations in five stages from "very bad condition" (level: 1) to "excellent condition" (level: 5).

As for "the number of times of operations", in one workday (it may extend over calendar days due to night work or the like) of each of the subjects, a period from an operation start instant to an operation end instant of the workday is counted as one time (one operation), and this period includes several-hour continuous driving, a break time, and so on. The estimation results by the biological state estimation device 100 are output once every about thirty minutes. Therefore, if one operation includes the continuous driving of one hour or longer, the estimation results are obtained a plurality of times from the biological state estimation device 100. On the other hand, the subjective evaluation by each of the subjects on the operation day is declared once at an instant when one operation is finished.

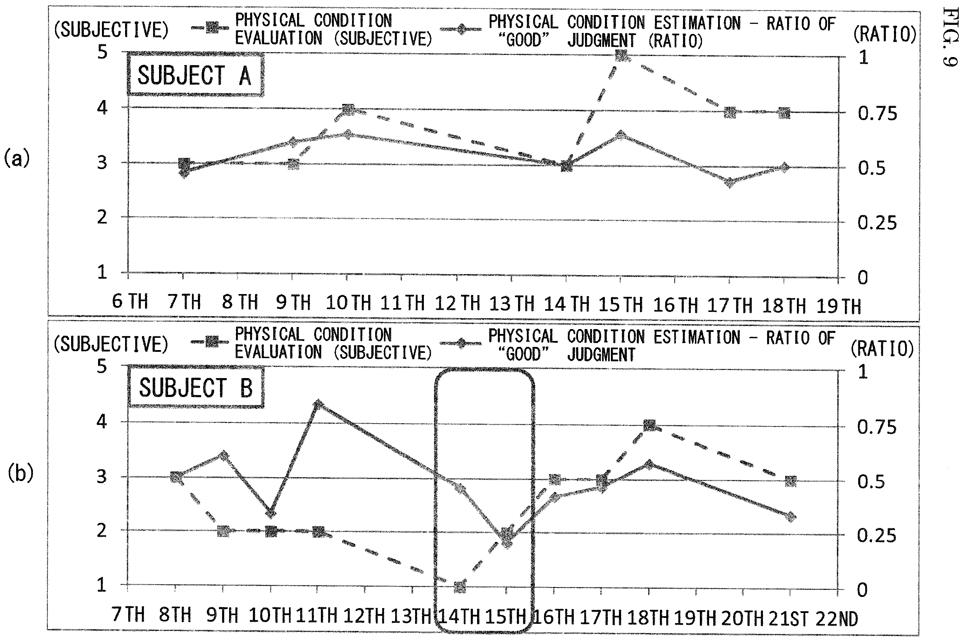

FIGs. 9(a), (b) illustrate the results of subjects A and B in a given month. In these drawings, a ratio of "good" estimation to the total number of judgment results in the physical condition estimation made by the biological state estimation device 100 during one operation (right vertical axis of each graph) and the five-stage subjective physical condition evaluation by each of the subjects after the operations of the days finished (left vertical axis of each graph) are plotted in a time-series manner for the respective operation days.

From FIGs. 9(a), (b), it is seen that in the data of both of the subjects, an increase/decrease tendency of a change of the ratio of the "good" judgment by the biological state estimation device 100 and a change tendency of the subjective evaluation are approximate to each other. Table 12 shows the comparison of the result of the physical condition estimation and the subjective evaluation by the subject A with those on the previous day (previous operation), and as compared with the previous day, a case where the ratio of the "good" physical condition estimation increases and a case where the subjective evaluation becomes better are indicated by "+", and a case where the ratio of the "good" physical condition estimation decreases and a case where the subjective evaluation becomes worse are indicated by "-", and as compared with the previous day, a case where the ratio of the "good" physical condition estimation is the same and a case where the subjective evaluation is the same are indicated by "±". Further, a case where the increasing and decreasing tendencies are the same is indicated by ○.

**[Table 12]**

| Date | 7th | 9th | 10th | 14th | 15th | 17th | 18th |
|---|---|---|---|---|---|---|---|
| Ratio of "good" judgment in physical condition estimation | | + | + | - | + | - | + |
| Physical condition evaluation (subjective) | | ± | + | - | + | - | ± |
| Agreement in increasing/decreasing tendency | | - | ○ | ○ | ○ | ○ | - |

According to Table 12, it is indicated that the subjectivity and the physical condition estimation tend to be approximate to each other, except on the days when the subjective evaluation is the same as that on the previous day.

In Table 13, which shows the summary of the results of all the subjects on which the comparison with the previous day (previous operation) similar to that in Table 12 was conducted, an agreement ratio of the increase/decrease tendency of all the seven subjects was 0.71 and the result of a Fisher's exact test was p = 0.032, and a significant correlation was recognized between an increase/decrease of the ratio of the "good" physical condition estimation by the biological state estimation device 100 and the subjective evaluation. Further, it turned out that the number of data in which the subjective evaluation is "worse" when the ratio of the "good" physical condition estimation "increases" is significantly small, and the number of data in which the subjective evaluation is "worse" when the ratio of the "good" physical condition estimation "decreases" is significantly large. On the other hand, the number of answers saying that the subjective evaluation is the same as that in the operation on the previous day is 32 out of the 71 operations, and the number of answers saying that the subjective evaluation is "normal (level: 3)" is 38 out of the 71 operations, which indicates that it is relatively difficult for the subjects to strictly realize daily changes in their physical conditions.

**[Table 13]**

| Evaluation comparison with previous operation | | Physical condition estimation | |
|---|---|---|---|
| | | Increase (+) | Decrease (-) |
| Subjective | Better (+) | 12 | 5 |
| | Worse (-) | 4 | 11 |

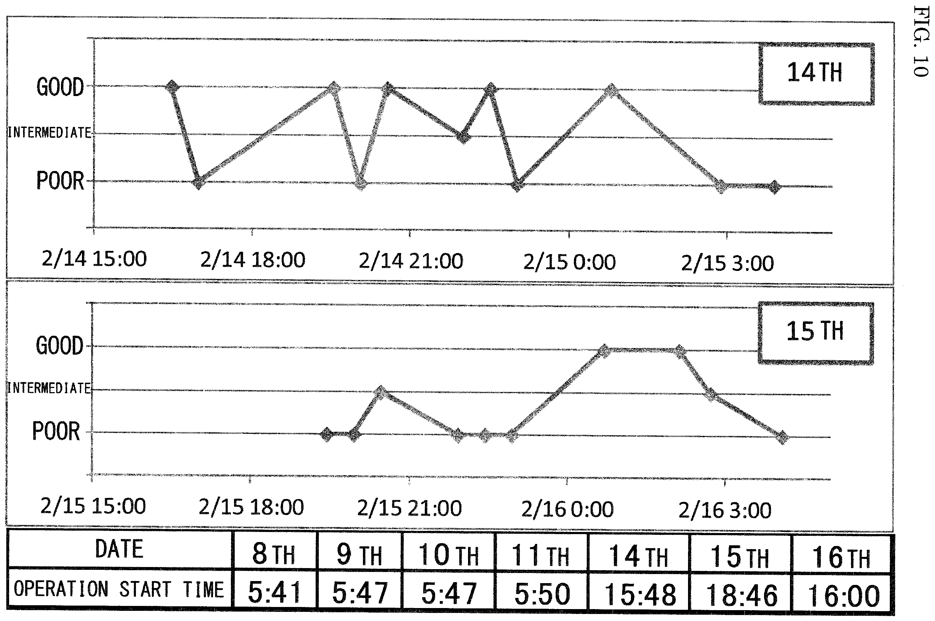

Here, in data of the subject B in FIG. 9(b), let us focus on the operations on the 14th for which the answer says the subjective evaluation is 1 (very bad condition) and the 15th. FIGs. 10 illustrate time-series data of the physical condition estimation results of the subject B on the 14th and the 15th, estimated by the biological state estimation device 100, and the operation start times on the 8th to the 16th. As for the operation on the 14th, the operation start time was 3:48 p.m. and the operation end time was past 3 in the morning on the 15th, and as for the operation on the 15th, the operation start time was 6:46 p.m. and the operation end time was past 3 in the morning on the 16th.

On the 14th, the physical condition estimation result fluctuates between "good" and "poor", and the "poor" estimation continues in the end. The subject B answered in a questionnaire on the 14th after the operation that he had felt slightly sleepy in the operation final stage, and from this it is inferred that he felt sleepiness and a poor physical condition because his work shift changed to an afternoon/night shift on the 14th from an early morning/day shift which had continued up to the 11th, and he was off on the 12th and the 13th, and because of these, his body could not sufficiently adapt and switch to a work mode and a night shift mode. On the 15th, the "poor" estimation was mostly made in the first half, but the "good" estimation" was also made in the latter half, which corresponds to the fact that the subjective evaluation on the 15th changes to level: 2 (bad condition) from level: 1 (very bad condition) on the 14th. Therefore, the physical condition estimation result by the biological state estimation device 100 of this embodiment can be said as close to the subjective evaluation by the subject, and in addition, it is also indicated that by outputting the result once every about thirty minutes, it is possible to reflect a change of the physical condition that the subject himself cannot easily realize.

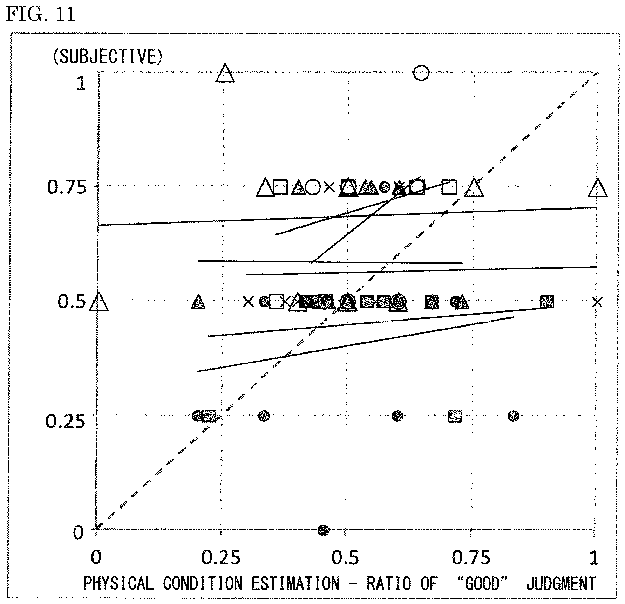

FIG. 11 is a distribution chart in which correlations between the subjective evaluations by all the subjects and the ratios of the "good" physical condition estimations by the physical condition estimation device 100 are normalized, and distribution approximations found for the respective subjects are represented by the thin lines. In FIG. 11, data located on a more right side than the broken line drawn obliquely at 45 degrees indicate that the ratio of the "good" physical condition estimation result is higher as compared with the subjective evaluation, and data located on a more left side than the broken line indicate that the ratio of the "good" physical condition estimation result is lower as compared with the subjective evaluation. Further, as a gradient of the straight line of the linear approximation for each subject is closer to 45 degrees, it is indicated that a positive correlation between the subjective evaluation and the physical condition estimation result is higher, as the gradient is closer to 0 degree, it is indicated that the correlation is lower, and when the gradient is minus, it is indicated that a negative correlation is high. The subjective evaluation is in five stages, while the physical condition estimation by the biological state estimation device 100 is in three stages of "good", "intermediate", and "poor", and accordingly the physical condition estimation result tends to spread in the left and right direction with respect to the subjective evaluation, but mostly the gradient is positive, and FIG. 11 also indicates that there is a certain degree of correlation between the physical condition estimation by the biological state estimation device 100 and the subjective evaluation.

In the above-described experiment examples, the computer program set in the biological state estimation device 100 being the in-vehicle computer and the data stored in its storage are used for the analysis, but it is also, of course, possible to perform the same analysis as above by setting these program and data in a computer of an operation manager. In this case, the analysis target data may be transmitted from the in-vehicle biological state estimation device 100 via a communication line (by radio or the like) to enable real-time analysis also in the computer of the operation manager, or the data stored in the in-vehicle biological state estimation device 100 may be taken out after the end of the driving work for ex-post analysis in the computer of the operation manager. By collecting such data, the operation manager can grasp the state of each driver during the driving, and in addition, can make use of these data for giving advice for more appropriate driving.

### Explanation of Reference Signs

- 1: biosignal measurement device
- 11: core pad
- 12: spacer pad
- 13: sensor
- 100: biological state estimation device
- 200: bioregulation function element judging means
- 210: frequency gradient time-series waveform calculating means
- 220: distribution ratio calculating means
- 230: fatigue curve calculating means
- 240: homeostasis function level calculating means
- 250: physical condition map calculating means
- 260: sensation map calculating means
- 300: basic physical condition estimating means
- 310: first basic physical condition estimating means
- 320: second basic physical condition estimating means

## Claims

1. A biological state estimation device which analyzes a biosignal measured from a person by a biosignal measurement device and estimates a biological state, the biological state estimation device comprising:
a bioregulation function element judging means which analyzes the biosignal and calculates a plurality of indexes indicating variations of states of bioregulation function elements, including an index ascribable to fluctuation highly correlated with a brain function, an autonomic nervous function, bodily and mental fatigue, or sensation, every predetermined judgment times set for the respective bioregulation function elements in advance to find time-series variations of the indexes;
a basic physical condition estimating means which estimates a basic physical condition of the person in a basic physical condition estimation time set longer than the judgment times of the bioregulation function elements in the bioregulation function element judging means; and
a basic physical condition outputting means which outputs a level of the basic physical condition of the person estimated by the basic physical condition estimating means, every time the predetermined basic physical condition estimation time passes,
wherein the basic physical condition estimating means estimates the basic physical condition of the person in the predetermined basic physical condition estimation time by analyzing the time-series variations relevant to the states of the bioregulation function elements which variations are found by the bioregulation function element judging means, in order of priorities set in advance, and referring to predetermined criteria.

2. The biological state estimation device according to claim 1,
wherein the basic physical condition estimating means includes:
a first basic physical condition estimating means which estimates that the basic physical condition of the person in the predetermined basic physical condition estimation time is on a predetermined level when the time-series variation of the bioregulation function element having a high priority, out of the time-series variations relevant to the bioregulation function elements which variations are found by the bioregulation function element judging means, satisfies a predetermined criterion; and
a second basic physical condition estimating means which, when the estimation is not made by the first basic physical condition estimating means, estimates that the basic physical condition of the person is on any of predetermined levels classified based on a predetermined criterion, by using the time-series variation relevant to another bioregulation function element having a lower priority than the priority of the bioregulation function element used in the first basic physical condition estimating means, and
wherein the basic physical condition outputting means outputs the level of the basic physical condition of the person estimated by the first basic physical condition estimating means or the second basic physical condition estimating means.

3. The biological state estimation device according to claim 2,
wherein, by using the time-series variation of the index highly correlated with the autonomic nervous function or the index highly correlated with the bodily and mental fatigue out of the plural bioregulation function elements on which the judgment is made by the bioregulation function element judging means, the first basic physical condition estimating means estimates that the level of the basic physical condition of the person is "good" or "poor" when the time-series variation satisfies a predetermined criterion.

4. The biological state estimation device according to claim 3, wherein the first basic physical condition estimating means estimates that the basic physical condition of the person is "poor" when the time-series variation of the index highly correlated with the autonomic nervous function satisfies a predetermined criterion, and estimates that the basic physical condition of the person is "good" when the time-series variation of the index highly correlated with the bodily and mental fatigue satisfies a predetermined criterion.

5. The biological state estimation device according to any one of claims 2 to 4, wherein, when the "good" or "poor" estimation is not made by the first basic physical condition estimating means, the second basic physical condition estimating means estimates the level of the basic physical condition of the person as one of "good", "poor", and "intermediate state" from the time-series variation of the index highly correlated with the sensation out of the plural bioregulation function elements on which the judgment is made by the bioregulation function element judging means.

6. A biological state estimation method which analyzes a biosignal measured from a person by a biosignal measurement device and estimates a biological state, by using a computer, the method comprising:
a bioregulation function element judging procedure which analyzes the biosignal and calculates a plurality of indexes indicating variations of states of bioregulation function elements, including an index ascribable to fluctuation highly correlated with a brain function, an autonomic nervous function, bodily and mental fatigue, or sensation, every predetermined judgment times set for the respective bioregulation function elements in advance to find time-series variations of the indexes;
a basic physical condition estimating procedure which estimates a basic physical condition of the person in a basic physical condition estimation time set longer than the judgment times of the bioregulation function elements in the bioregulation function element judging procedure; and
a basic physical condition outputting procedure which outputs a level of the basic physical condition of the person estimated by the basic physical condition estimating means, every time the predetermined basic physical condition estimation time passes,
wherein the basic physical condition estimating procedure estimates the basic physical condition of the person in the predetermined basic physical condition estimation time by analyzing the time-series variations relevant to the states of the bioregulation function elements which variations are found by the bioregulation function element judging procedure, in order of priorities set in advance, and referring e to predetermined criteria.

7. The biological state estimation method according to claim 6,
wherein the basic physical condition estimating procedure includes:
a first basic physical condition estimating procedure which estimates that the basic physical condition of the person in the predetermined basic physical condition estimation time is on a predetermined level when the time-series variation of the bioregulation function element having a high priority, out of the time-series variations relevant to the bioregulation function elements which variations are found by the bioregulation function element judging procedure, satisfies a predetermined criterion; and
a second basic physical condition estimating procedure which, when the estimation is not made by the first basic physical condition estimating procedure, estimates that the basic physical condition of the person is on any of predetermined levels classified based on a predetermined criterion, by using the time-series variation relevant to another bioregulation function element having a lower priority than the priority of the bioregulation function element used in the first basic physical condition estimating procedure, and
wherein the basic physical condition outputting procedure outputs the level of the basic physical condition of the person estimated by the first basic physical condition estimating procedure or the second basic physical condition estimating procedure.

8. A computer program causing a computer as a biological state estimation device to analyze a biosignal measured from a person by a biosignal measurement device and execute a biological state estimating procedure which estimates a biological state, the computer program causing the computer to execute, as the biological state estimating procedure:
a bioregulation function element judging procedure which analyzes the biosignal and calculates a plurality of indexes indicating variations of states of bioregulation function elements, including an index ascribable to fluctuation highly correlated with a brain function, an autonomic nervous function, bodily and mental fatigue, or sensation, every predetermined judgment times set for the respective bioregulation function elements in advance to find time-series variations of the indexes;
a basic physical condition estimating procedure which estimates a basic physical condition of the person in a basic physical condition estimation time set longer than the judgment times of the bioregulation function elements in the bioregulation function element judging procedure; and
a basic physical condition outputting procedure which outputs a level of the basic physical condition of the person estimated by the basic physical condition estimating procedure, every time the predetermined basic physical condition estimation time passes,
wherein the basic physical condition estimating procedure estimates the basic physical condition of the person in the predetermined basic physical condition estimation time by analyzing the time-series variations relevant to the states of the bioregulation function elements which variations are found by the bioregulation function element judging procedure, in order of priorities set in advance, and referring to predetermined criteria.

9. The computer program according to claim 8,
wherein the basic physical condition estimating procedure executes:
a first basic physical condition estimating procedure which estimates that the basic physical condition of the person in the predetermined basic physical condition estimation time is on a predetermined level when the time-series variation of the bioregulation function element having a high priority, out of the time-series variations relevant to the bioregulation function elements which variations are found by the bioregulation function element judging procedure, satisfies a predetermined criterion; and
a second basic physical condition estimating procedure which, when the estimation is not made by the first basic physical condition estimating procedure, estimates that the basic physical condition of the person is on any of predetermined levels classified based on a predetermined criterion, by using the time-series variation relevant to another bioregulation function element having a lower priority than the priority of the bioregulation function element used in the first basic physical condition estimating procedure, and
wherein the basic physical condition outputting procedure outputs the level of the basic physical condition of the person estimated by the first basic physical condition estimating procedure or the second basic physical condition estimating procedure.

10. The computer program according to claim 9,
wherein, by using the time-series variation of the index highly correlated with the autonomic nervous function or the index highly correlated with the bodily and mental fatigue out of the plural bioregulation function elements on which the judgment is made by the bioregulation function element judging procedure, the first basic physical condition estimating procedure estimates that the level of the basic physical condition of the person is "good" or "poor" when the time-series variation satisfies a predetermined criterion.

11. The computer program according to claim 9 or 10, wherein, when the "good" or "poor" estimation is not made by the first basic physical condition estimating procedure, the second basic physical condition estimating procedure estimates the level of the basic physical condition of the person as one of "good", "poor", and "intermediate state" from the time-series variation of the index highly correlated with the sensation out of the plural bioregulation function elements on which the judgment is made by the bioregulation function element judging procedure.
